# EUROPEAN PATENT SPECIFICATION

(11) **EP 2 932 941 B1**
(45) Date of publication and mention of the grant of the patent: **18.09.2019**
(21) Application number: 15167203.7
(22) Date of filing: 06.12.2005
(51) Int. Cl.: A61F 2/28, A61B 18/00, A61L 24/02, A61L 27/50, A61F 2/44, A61B 17/70

(54) **BONE TREATMENT SYSTEMS**
KNOCHENBEHANDLUNGSSYSTEME
SYSTÈMES DE TRAITEMENT OSSEUX

(30) Priority: 06.12.2004 US 633509 P; 24.06.2005 US 165651; 24.06.2005 US 165652; 02.08.2005 US 196089; 02.08.2005 US 196045; 20.08.2005 US 208448; 22.08.2005 US 209035
(43) Date of publication of application: 21.10.2015
(62) Divisional of application: 11007504.1
(73) Proprietor: Dfine Inc., South Jordan, UT 84095 (US)
(72) Inventor: Truckai, Csaba, Saratoga CA California 95070 (US); Shadduck, John, Tuburon CA California 94920 (US); Luzzi, Robert, Pleasanton CA California 94114 (US); Carlson, Grace, San Francisco CA California 94122 (US); Rappleye, Travis, San Jose CA California 95123 (US)
(74) Representative: Bradley, Adrian

(56) References cited:
- WO-A-02/087416
- US-A1- 2002 156 483
- US-A1- 2004 193 171

## Description

The present invention relates in certain embodiments to medical devices for treating osteoplasty procedures such as vertebral compression fractures. More particularly, embodiments of the invention relate to instruments and methods for controllably restoring vertebral body height by controlling bone fill material flows into the interior of a vertebra. In one embodiment, a system utilizes Rf energy in combination a conductive bone fill material and a controller for controlling bone fill material flow parameters and energy delivery parameters for selectively polymerizing the surface of the inflow plume to control the geometry of the fill material and the application of force caused by inflows of fill material or to provide a pulsed bone fill material flow with high acceleration rates for applying expansion forces to the bone and for controlling the geometry of the bone fill material.

### Description of the Related Art

Osteoporotic fractures are prevalent in the elderly, with an annual estimate of 1.5 million fractures in the United States alone. These include 750,000 vertebral compression fractures (VCFs) and 250,000 hip fractures. The annual cost of osteoporotic fractures in the United States has been estimated at $13.8 billion. The prevalence of VCFs in women age 50 and older has been estimated at 26%. The prevalence increases with age, reaching 40% among 80-year-old women. Medical advances aimed at slowing or arresting bone loss from aging have not provided solutions to this problem. Further, the population affected will grow steadily as life expectancy increases. Osteoporosis affects the entire skeleton but most commonly causes fractures in the spine and hip. Spinal or vertebral fractures also cause other serious side effects, with patients suffering from loss of height, deformity and persistent pain which can significantly impair mobility and quality of life. Fracture pain usually lasts 4 to 6 weeks, with intense pain at the fracture site. Chronic pain often occurs when one vertebral level is greatly collapsed or multiple levels are collapsed.

Postmenopausal women are predisposed to fractures, such as in the vertebrae, due to a decrease in bone mineral density that accompanies postmenopausal osteoporosis. Osteoporosis is a pathologic state that literally means "porous bones". Skeletal bones are made up of a thick cortical shell and a strong inner meshwork, or cancellous bone, of with collagen, calcium salts and other minerals. Cancellous bone is similar to a honeycomb, with blood vessels and bone marrow in the spaces. Osteoporosis describes a condition of decreased bone mass that leads to fragile bones which are at an increased risk for fractures. In an osteoporosis bone, the sponge-like cancellous bone has pores or voids that increase in dimension making the bone very fragile. In young, healthy bone tissue, bone breakdown occurs continually as the result of osteoclast activity, but the breakdown is balanced by new bone formation by osteoblasts. In an elderly patient, bone resorption can surpass bone formation thus resulting in deterioration of bone density. Osteoporosis occurs largely without symptoms until a fracture occurs.

Vertebroplasty and kyphoplasty are recently developed techniques for treating vertebral compression fractures. Percutaneous vertebroplasty was first reported by a French group in 1987 for the treatment of painful hemangiomas. In the 1990's, percutaneous vertebroplasty was extended to indications including osteoporotic vertebral compression fractures, traumatic compression fractures, and painful vertebral metastasis. Vertebroplasty is the percutaneous injection of PMMA (polymethylmethacrylate) into a fractured vertebral body via a trocar and cannula. The targeted vertebrae are identified under fluoroscopy. A needle is introduced into the vertebrae body under fluoroscopic control, to allow direct visualization. A bilateral transpedicular (through the pedicle of the vertebrae) approach is typical but the procedure can be done unilaterally. The bilateral transpedicular approach allows for more uniform PMMA infill of the vertebra.

In a bilateral approach, approximately 1 to 4 ml of PMMA is used on each side of the vertebra. Since the PMMA needs to be is forced into the cancellous bone, the techniques require high pressures and fairly low viscosity cement. Since the cortical bone of the targeted vertebra may have a recent fracture, there is the potential of PMMA leakage. The PMMA cement contains radiopaque materials so that when injected under live fluoroscopy, cement localization and leakage can be observed. The visualization of PMMA injection and extravasion are critical to the technique-and the physician terminates PMMA injection when leakage is evident. The cement is injected using syringes to allow the physician manual control of injection pressure.

Kyphoplasty is a modification of percutaneous vertebroplasty. Kyphoplasty involves a preliminary step consisting of the percutaneous placement of an inflatable balloon tamp in the vertebral body. Inflation of the balloon creates a cavity in the bone prior to cement injection. The proponents of percutaneous kyphoplasty have suggested that high pressure balloon-tamp inflation can at least partially restore vertebral body height. In kyphoplasty, some physicians state that PMMA can be injected at a lower pressure into the collapsed vertebra since a cavity exists, when compared to conventional vertebroplasty.

The principal indications for any form of vertebroplasty are osteoporotic vertebral collapse with debilitating pain. Radiography and computed tomography must be performed in the days preceding treatment to determine the extent of vertebral collapse, the presence of epidural or foraminal stenosis caused by bone fragment retropulsion, the presence of cortical destruction or fracture and the visibility and degree of involvement of the pedicles.

Leakage of PMMA during vertebroplasty can result in very serious complications including compression of adjacent structures that necessitate emergency decompressive surgery. See "Anatomical and Pathological Considerations in Percutaneous Vertebroplasty and Kyphoplasty: A Reappraisal of the Vertebral Venous System", Groen, R. et al, Spine Vol. 29, No. 13, pp 1465-1471 2004. Leakage or extravasion of PMMA is a critical issue and can be divided into paravertebral leakage, venous infiltration, epidural leakage and intradiscal leakage. The exothermic reaction of PMMA carries potential catastrophic consequences if thermal damage were to extend to the dural sac, cord, and nerve roots. Surgical evacuation of leaked cement in the spinal canal has been reported. It has been found that leakage of PMMA is related to various clinical factors such as the vertebral compression pattern, and the extent of the cortical fracture, bone mineral density, the interval from injury to operation, the amount of PMMA injected and the location of the injector tip. In one recent study, close to 50% of vertebroplasty cases resulted in leakage of PMMA from the vertebral bodies. See Hyun-Woo Do et al, "The Analysis of Polymethylmethacrylate Leakage after Vertebroplasty for Vertebral Body Compression Fractures", Jour. of Korean Neurosurg. Soc. Vol. 35, No. 5 (5/2004) pp. 478-82, (http://www.jkns.or.kr/htm/abstract.asp?no=0042004086).

Another recent study was directed to the incidence of new VCFs adjacent to the vertebral bodies that were initially treated. Vertebroplasty patients often return with new pain caused by a new vertebral body fracture. Leakage of cement into an adjacent disc space during vertebroplasty increases the risk of a new fracture of adjacent vertebral bodies. See Am. J. Neuroradiol. 2004 Feb; 25(2):175-80. The study found that 58% of vertebral bodies adjacent to a disc with cement leakage fractured during the follow-up period compared with 12% of vertebral bodies adjacent to a disc without cement leakage.

Another life-threatening complication of vertebroplasty is pulmonary embolism. See Bernhard, J. et al, "Asymptomatic diffuse pulmonary embolism caused by acrylic cement: an unusual complication of percutaneous vertebroplasty", Ann. Rheum. Dis. 2003; 62:85-86. The vapors from PMMA preparation and injection also are cause for concern. See Kirby, B, et al., "Acute bronchospasm due to exposure to polymethylmethacrylate vapors during percutaneous vertebroplasty", Am. J. Roentgenol. 2003; 180:543-544.

In both higher pressure cement injection (vertebroplasty) and balloon-tamped cementing procedures (kyphoplasty), the methods do not provide for well controlled augmentation of vertebral body height. The direct injection of bone cement simply follows the path of least resistance within the fractured bone. The expansion of a balloon applies also compacting forces along lines of least resistance in the collapsed cancellous bone. Thus, the reduction of a vertebral compression fracture is not optimized or controlled in high pressure balloons as forces of balloon expansion occur in multiple directions.

In a kyphoplasty procedure, the physician often uses very high pressures (e.g., up to 200 or 300 psi) to inflate the balloon which crushes and compacts cancellous bone. Expansion of the balloon under high pressures close to cortical bone can fracture the cortical bone, typically the endplates, which can cause regional damage to the cortical bone with the risk of cortical bone necrosis. Such cortical bone damage is highly undesirable as the endplate and adjacent structures provide nutrients for the disc.

Kyphoplasty also does not provide a distraction mechanism capable of 100% vertebral height restoration. Further, the kyphoplasty balloons under very high pressure typically apply forces to vertebral endplates within a central region of the cortical bone that may be weak, rather than distributing forces over the endplate.

There is a general need to provide systems and methods for use in treatment of vertebral compression fractures that provide a greater degree of control over introduction of bone support material, and that provide better outcomes. The present invention meets this need and provides several other advantages in a novel and nonobvious manner.

### SUMMARY OF THE INVENTION

The invention provides a system for treating bone according to claim 1. Also provided are systems and methods for utilizing Rf energy in combination with a bone fill material that carries an electrically conductive filler. In some embodiments a controller controls cement delivery parameters and energy delivery parameters for selectively polymerizing surface portions of the inflow plume to thereby control the direction of flow and the ultimate geometry of a flowable, in-situ hardenable composite. The system and method may further include means for sealing tissue in the interior of a vertebra to prevent migration of monomers, fat or emboli into the patient's bloodstream. Certain embodiments of the invention provide a bone fill material that allows for controlled sealing or coagulation of tissue in a targeted region of cancellous bone. Other embodiments of the invention provide a bone fill material for vertebral compression fractures that delivers osteoinductive agents, growth factors and chemotherapeutic agents for enhancing bone ingrowth.

A method for treating bone is provided. The method comprises introducing an infill material into an interior region of a bone and contemporaneously applying energy from an energy source as the infill material is introduced to selectively alter a flowability property of the infill material.

Another method for treating bone is provided. The method comprises providing a bone fill material including a composition that has at least one of an energy-absorbing property and an energy-transmitting property.
The method also comprises introducing a volume of the fill material into an interior of a bone member and applying energy from a remote energy source to the composition, wherein the energy-absorbing or energy-transmitting property thereby causes a thermal modification in at least one of the fill material and adjacent portions of the interior of the bone member.

Another method for treating bone is provided comprising introducing at least one instrument working end into an interior region of a bone member. The method also comprises introducing an infill material into the interior region of the bone member and applying energy from an energy source through the at least one working end to the fill material to seal tissue about the surface of the fill material, wherein the sealed tissue prevents migration of emboli into the a blood stream.

Another method for treating bone is provided. The method comprises introducing a volume of polymerizable bone fill material into a bone. The method also comprises altering a property of at least a portion of the volume to create a differential in flowability wherein a first selected region is substantially non-flowable and a second selected region is flowable. The method also comprises introducing an additional volume of fill material wherein a direction of flow of the additional volume is controlled by said differential in flowability.

Another method for controlling the direction of flow of a bone fill material in a vertebra is provided. The method comprises injecting a volume of bone filler into a vertebra and selectively modifying the viscosity of a selected portion of the fill material to control a direction of flow, wherein said modification defines at least one higher viscosity portion and at least one lower viscosity portion of the fill material.

Another method for treating a vertebral body is provided comprising introducing a bone fill material into an interior of the vertebral body, the bone fill material interdigitating with cancellous bone. The method also comprises increasing the viscosity of the bone fill material at selected regions within the interior of the vertebral body to form substantially flow impermeable portions within the vertebral body, and applying an expansion force within the bone fill material.

Another method for moving a body structure is provided. The method comprises introducing a flowable material into an interior of the body structure. The method also comprises selectively transforming a selected portion of the flowable material in the interior of the body structure into an elastic portion, said elastic portion at least partially surrounded by body structure, and applying an expansion force within said elastic portion thereby expanding the elastic portion to move the body structure.

The system comprises an elongated introducer having a working end with a flow channel extending therethrough for injecting fill material into an interior of a bone. A source of fill material is coupled to the flow channel, wherein the fill material may include a composition that has at least one energy-absorbing property and an energy-transmitting property. An energy source may be configured to apply energy to the energy-absorbing or energy-transmitting composition to modify at least one of the fill material and a bone structure proximate the injected fill material.

In accordance with another embodiment, an osteoplasty system is provided. The osteoplasty system comprises an elongated introducer with a flow channel extending therethrough for introducing a fill material to a bone portion. A pressurizable source of fill material is coupled to the flow channel, wherein the fill material includes an electrically conductive filler. A radiofrequency (Rf) source is operatively connected to at least one electrode in a working end of the introducer for delivering Rf energy to the fill material.

In accordance with another embodiment, a bone fill material is provided comprising an in-situ hardenable component and an electrically conductive filler component that enables the bone fill material to function as an electrode.

In accordance with another embodiment, a bone fill material is provided comprising a composite including an in-situ hardenable component and a filler component. The filler component has at least one of an energy-absorbing property and an energy-transmitting property for cooperating with a remote energy source for absorbing energy for polymerizing the composite or for transmitting energy for heating tissue adjacent the composite.

In accordance with another embodiment, a bone fill material is provided comprising an in-situ hardenable cement component. The bone fill material also comprises an electrically conductive filler component comprising a biocompatible conductive metal, wherein the filler component comprises microfilaments enabling the bone fill material to function as an electrode.

Another method for treating a body is provided. The method comprises advancing an introducer into the vertebral body and injecting bone fill material from the introducer into the vertebral body. The method also comprises applying Rf energy to the bone fill material at a distal portion of the introducer, the Rf energy causing the viscosity of the bone fill material to increase at said distal portion such that the bone fill material exits the introducer at said increased viscosity.

Another method for treating an abnormal vertebra is provided. The method comprises providing a flow source configured to provide a bone fill material inflow into a vertebral body. The method also comprises inflowing bone fill material into the vertebral body, measuring a characteristic of the bone fill material, and adjusting an inflow parameter of the bone fill material inflow in response to the measured characteristic of the bone fill material.

Another method of treating an abnormal vertebra is provided. The method comprises advancing an introducer into a vertebral body, injecting bone fill material through the introducer into the vertebral body, and measuring a characteristic of the bone fill material with a sensor while the bone fill material is being injected into the vertebral body. The method also comprises adjusting an inflow parameter of the bone fill material in response to the measured characteristic of the bone fill material, and continuing the injection of bone fill material through the introducer into the vertebral body using the adjusted inflow parameter.

In accordance with another embodiment, a system for treating an abnormal vertebra is provided. The system comprises an elongated introducer configured for insertion into a vertebra and a source of flowable bone fill material coupled to the introducer, the source configured to provide a pressurized flow of bone fill material into the vertebra through a channel of the introducer. The system also comprises a sensor configured to measure a characteristic of the bone fill material and a controller configured to control at least one flow parameter of the bone fill material flow in response to the measured characteristic of the bone fill material.

Another method of treating an abnormal vertebra is provided. The method comprises flowing a volume of bone fill material into a vertebral body under flow parameters sufficient to allow at least a portion of the volume of bone fill material to interdigitate with cancellous bone. The method also comprises adjusting at least one of said flow parameters so that flowing an additional volume of bone fill material into the vertebral body substantially prevents interdigitation of the bone fill material with cancellous bone, the additional volume of bone fill material thereby applying forces to the cancellous bone and interdigitated fill material about a surface of the bone fill material.

Another method of treating an abnormal vertebra is provided. The method comprises providing a flow source configured to provide bone fill material inflows into an interior of a vertebra through an introducer and a controller configured to control the bone fill material inflows, and inflowing the bone fill material in pulses into the interior of the vertebra.

Another method of treating an abnormal vertebra is provided. The method comprises injecting a flow of bone fill material into a vertebral body, said flow being injected under parameters sufficient to allow the flow to interdigitate with cancellous bone. The method also comprises continuing the flow of bone fill material into the vertebral body, wherein the continued flow expands the cancellous bone with the interdigitated bone fill material.

In accordance with another embodiment, a system for treating an abnormal vertebra is provided. The system comprises an elongated introducer configured for insertion into a vertebra and a source of flowable bone fill material coupled to the introducer, the source configured to provide a pressurized flow of bone fill material through the introducer. The system also comprises a controller configured to control at least one flow parameter of the bone fill material into the vertebra, the controller being adjustable between a first setting wherein the bone fill material is capable of interdigitating with cancellous bone and a second setting wherein the bone fill material is substantially prevented from interdigitating with cancellous bone.

These and other objects of the present invention will become readily apparent upon further review of the following drawings and specification.

### BRIEF DESCRIPTION OF THE DRAWINGS

In order to better understand the invention and to see how it may be carried out in practice, some preferred embodiments are next described, by way of nonlimiting examples only, with reference to the accompanying drawings, in which like reference characters denote corresponding features consistently throughout similar embodiments in the attached drawings.
FIG. 1 is a schematic side view of a spine segment showing a vertebra with a compression fracture and an introducer, in accordance with one embodiment disclosed herein.
FIG. 2A is a schematic perspective view of a system for treating bone, in accordance with one embodiment.
FIG. 2B is a schematic perspective sectional view of a working end of the introducer taken along line 2B-2B of FIG. 2A.
FIG. 2C is a schematic view of a screw pump for use in one embodiment of an introducer to deliver fill material into the interior of a vertebra.
FIG. 3A is a schematic perspective view of a working end of a probe, in accordance with one embodiment.
FIG. 3B is a schematic perspective view of a working end of a probe, in accordance with another embodiment.
FIG. 3C is a schematic perspective view of a working end of a probe, in accordance with yet another embodiment.
FIG. 4 is a schematic sectional side view of one embodiment of a working end of a probe, in accordance with one embodiment.
FIG. 5A is a schematic side view of a probe inserted into a vertebral body and injecting flowable fill material into the vertebral body.
FIG. 5B is a schematic side view of the probe in FIG. 5A injecting a relatively high viscosity volume of flowable fill material into the vertebral body, in accordance with one embodiment of the present invention.
FIG. 6 is a schematic perspective view of a system for treating bone, in accordance with another embodiment.
FIG. 7A is a schematic sectional view of a fill material, in accordance with one embodiment.
FIG. 7B is a schematic sectional view of a fill material, in accordance with another embodiment.
FIG. 8A is a schematic perspective view of a system for treating bone, in accordance with another embodiment.
FIG. 8B is a schematic perspective view of the system in FIG. 8A, injecting an additional volume of fill material into a vertebral body.
FIG. 9A is a schematic sectional view of one step in a method for treating bone, in accordance with one embodiment.
FIG. 9B is a schematic sectional view of another step in a method for treating bone, in accordance with one embodiment.
FIG. 9C is a schematic sectional view of still another step in a method for treating bone, in accordance with one embodiment.
FIG. 10A is a schematic sectional view of a step in a method for treating bone, in accordance with another embodiment.
FIG. 10B is a schematic sectional view of another step in a method for treating bone, in accordance with another embodiment.
FIG. 11A is a schematic perspective view of a system for treating bone, in accordance with another embodiment.
FIG. 11B is a schematic perspective view of the system in FIG. 11A, applying energy to a fill material.
FIG. 12 is a schematic perspective view of a system for treating bone, in accordance with another embodiment.
FIG. 13A is a side view of a working end of an introducer, in accordance with one embodiment.
FIG. 13B is a side view of a working end of an introducer, in accordance with another embodiment.
FIG. 13C is a side view of a working end of an introducer, in accordance with yet another embodiment.
FIG. 13D is a side view of a working end of an introducer, in accordance with still another embodiment.
FIG. 14 is a perspective view of a system for treating bone, in accordance with another embodiment.
FIG. 15 is a schematic view of another embodiment of bone fill material delivery system together with an aspiration source and a working end of an introducer, in accordance with one embodiment.
FIG. 16A is a sectional view of a working end of an introducer, as in FIG. 15 showing the orientation of a cement injection port in a vertebra.
FIG. 16B is a sectional view of the working end of FIG. 16A showing an initial inflow of bone cement.
FIG. 16C is a sectional view of the working end of FIG. 16B showing an additional inflow of bone cement to reduces a vertebral fracture.
FIG. 17A is a sectional view of a vertebra depicting a first mode of operation wherein an initial flow of bone cement is provided under selected flow parameters that allow cement interdigitation into cancellous bone.
FIG. 17B is a sectional view of a vertebra similar to FIG. 17A depicting a second mode of operation wherein cement flows are provided in a high acceleration pulse that disallows cement interdigitation into cancellous bone.

### DETAILED DESCRIPTION OF THE PREFERRED EMBODIMENTS

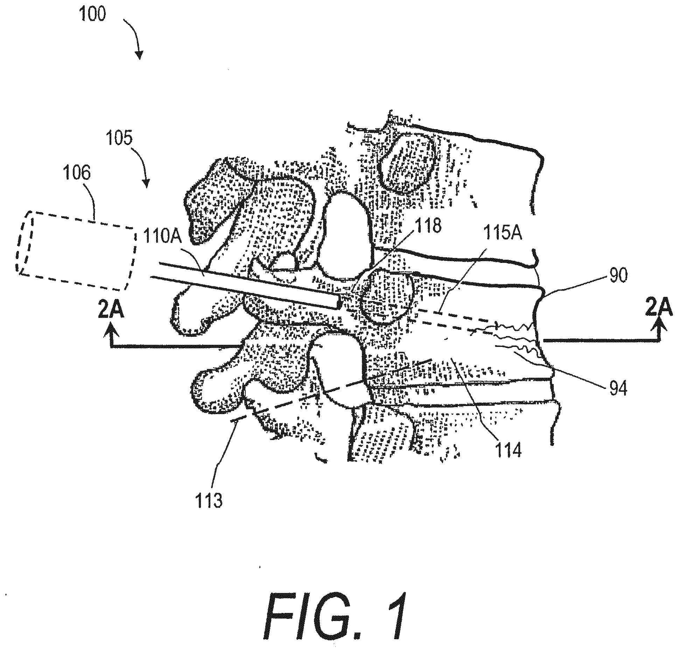

FIG. 1 illustrates one embodiment of the invention for treating a spine segment in which a vertebral body **90** has a wedge compression fracture indicated at **94.** In one embodiment, the systems and methods of the invention are directed to safely introducing a bone fill material into cancellous bone of the vertebra without extravasion of fill material in unwanted directions (i) to prevent micromotion in the fracture for eliminating pain, and (ii) to support the vertebra and increase vertebral body height. Further, the invention includes systems and methods for sealing cancellous bone (e.g., blood vessels, fatty tissues etc.) in order to prevent monomers, fat, fill material and other emboli from entering the venous system during treatment.

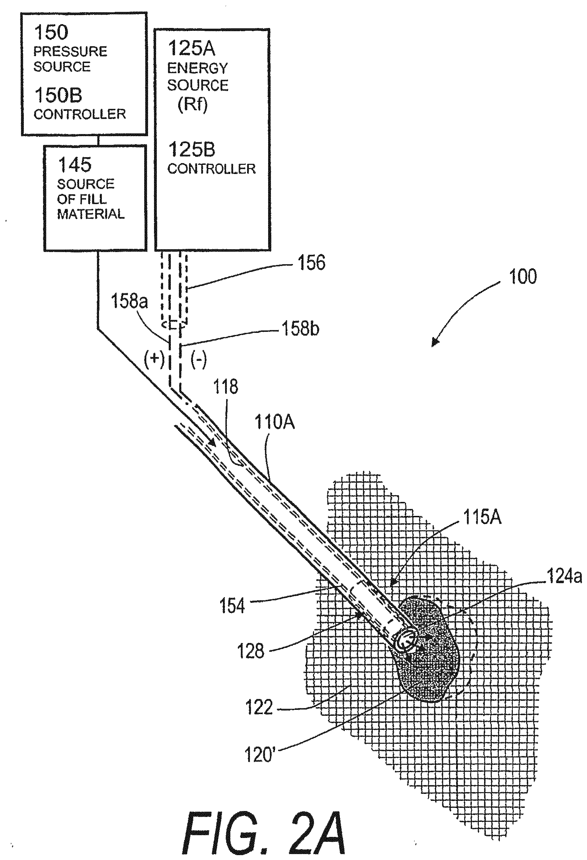

FIG. 1 illustrates a fractured vertebra and bone infill system **100** which includes a probe **105** having a handle end **106** extending to an elongated introducer **110A** and working end **115A,** shown in FIG. 2A. The introducer is shown introduced through pedicle **118** of the vertebra for accessing the osteoporotic cancellous bone **122** (See Fig 2A). The initial aspects of the procedure are similar to conventional percutaneous vertebroplasty wherein the patient is placed in a prone position on an operating table. The patient is typically under conscious sedation, although general anesthesia is an alternative. The physician injects a local anesthetic (e.g., 1% Lidocaine) into the region overlying the targeted pedicle or pedicles as well as the periosteum of the pedicle(s). Thereafter, the physician uses a scalpel to make a 1 to 5 mm skin incision over each targeted pedicle. Thereafter, the introducer **110A** is advanced through the pedicle into the anterior region of the vertebral body, which typically is the region of greatest compression and fracture. The physician confirms the introducer path posterior to the pedicle, through the pedicle and within the vertebral body by anteroposterior and lateral X-Ray projection fluoroscopic views. The introduction of infill material as described below can be imaged several times, or continuously, during the treatment depending on the imaging method.

It should be appreciated that the introducer also can be introduced into the vertebra from other angles, for example, along axis **113** through the wall of the vertebral body **114** as in FIG. 1 or in an anterior approach (not shown). Further, first and second cooperating introducers can be used in a bilateral transpedicular approach. Additionally, any mechanism known in the art for creating an access opening into the interior of the vertebral body 90 can be used, including open surgical procedures.

### DEFINITIONS

"Bone fill material, infill material or composition" includes its ordinary meaning and is defined as any material for infilling a bone that includes an in-situ hardenable material. The fill material also can include other "fillers" such as filaments, microspheres, powders, granular elements, flakes, chips, tubules and the like, autograft or allograft materials, as well as other chemicals, pharmacological agents or other bioactive agents.

"Flowable material" includes its ordinary meaning and is defined as a material continuum that is unable to withstand a static shear stress and responds with an irrecoverable flow (a fluid)-unlike an elastic material or elastomer that responds to shear stress with a recoverable deformation. Flowable material includes fill material or composites that include a fluid (first) component and an elastic or inelastic material (second) component that responds to stress with a flow, no matter the proportions of the first and second component, and wherein the above shear test does not apply to the second component alone.

An "elastomer" includes its ordinary meaning and is defined as material having to some extent the elastic properties of natural rubber wherein the material resumes or moves toward an original shape when a deforming force is removed.

"Substantially" or "substantial" mean largely but not entirely. For example, substantially may mean about 10% to about 99.999%, about 25% to about 99.999% or about 50% to about 99.999%.

"Osteoplasty" includes its ordinary meaning and means any procedure wherein fill material is delivered into the interior of a bone.

"Vertebroplasty" includes its ordinary meaning and means any procedure wherein fill material is delivered into the interior of a vertebra.

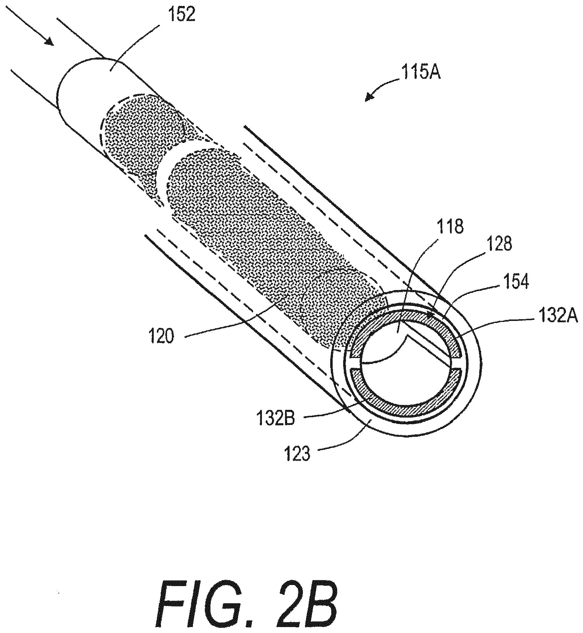

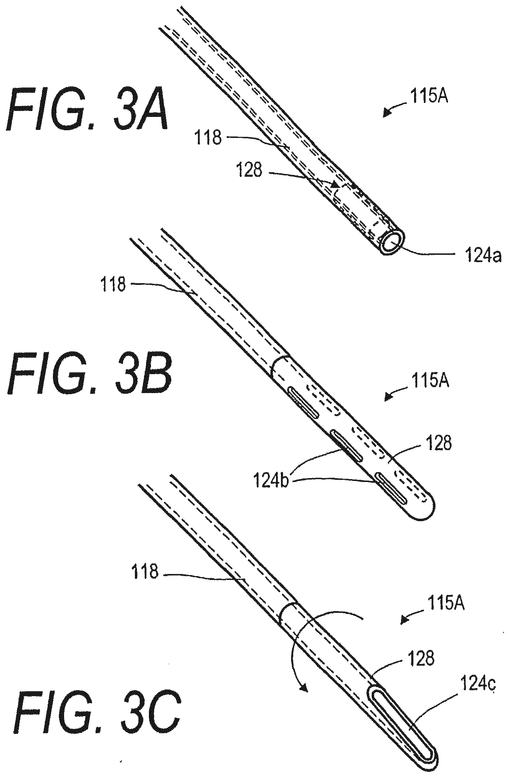

Now referring to FIGS. 2A and 2B, the end of introducer **110A** is shown schematically after being introduced into cancellous bone **122** with an inflow of fill material indicated at **120.** The cancellous bone can be in any bone, for example in a vertebra. It can be seen that the introducer **110A** and working end **115A** comprise a sleeve or shaft that is preferably fabricated of a metal having a flow channel **118** extending therethrough from the proximal handle end **106** (see FIG. 1). In one embodiment, the introducer shaft is a stainless steel tube **123** having an outside diameter ranging between about 3.5 and 4.5 mm, but other dimensions are possible. In another embodiment, the sleeve or shaft is fabricated of plastic. As can be seen in FIG. 2A and 3A, the flow channel **118** can terminate in a single distal open termination or outlet **124a** in the working end **115A,** or there can be a plurality of flow outlets or ports **124b** configured angularly about the radially outward surfaces of the working end **115A** of FIG. 3B. The outlets in the working end thus allow for distal or radial ejection of fill material, or a working end can have a combination of radial and distal end outlets. As can be seen in FIG. 3C, the distal end of working end **115A** also can provide an angled distal end outlet **124c** for directing the flow of fill material from the outlet by rotating the working end.

In FIGS. 2A and 2B, it can be seen that system **100** includes a remote energy source **125A** and controller **125B** that are operatively coupled to an energy emitter **128** in working end **115A** for applying energy to fill material **120** contemporaneous with and subsequent to ejection of the fill material from the working end. As shown in FIG. 2A, a preferred energy source **125A** is a radiofrequency (Rf) source known in the art that is connected to at least one electrode **(132a** and **132b** in FIGS. 2A and 2B) in contact with injected fill material **120** that carries a radiosensitive composition therein. It is equally possible to use other remote energy sources and emitters **128** in the working end which fall within the scope of the invention, such as (i) an electrical source coupled to a resistive heating element in the working end, (ii) a light energy source (coherent or broadband) coupled to an optical fiber or other light channel terminating in the working end; (iii) an ultrasound source coupled to an emitter in the working end; or a (iv) or a microwave source coupled to an antenna in the working end. In still another embodiment, the energy source can be a magnetic source. The fill material is configured with an energy-absorbing material or an energy-transmitting material that cooperates with energy delivery from a selected energy source. For example, the energy-absorbing or energy-transmitting material can be a radiosensitive or conductive material for cooperating with an Rf source, chromophores for cooperating with a light source, ferromagnetic particles for cooperating with a magnetic source, and the like. In one embodiment, the fill material **120** can include a composition having an energy-absorbing property and an energy-transmitting property for cooperating with the remote energy source **125A.** For example, the composition can absorb energy from the remote energy source **125A** for polymerizing the composite or transmit energy for heating tissue adjacent to the composite.

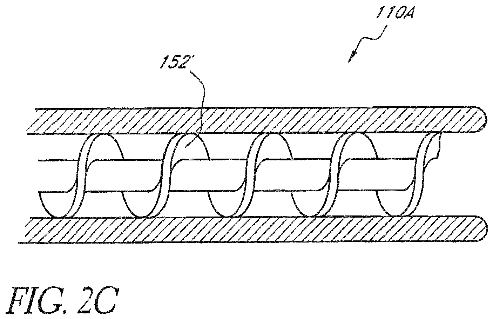

As can be understood from FIGS. 2A and 2B, the introducer **110A** is operatively coupleable to a source **145** of bone fill material **120** together with a pressure source or mechanism **150** that operates on the source of fill material to deliver the fill material **120** through the introducer **110A** into a bone (see arrows). The pressure source **150** can comprise any type of pump mechanism, such as a piston pump, screw pump or other hydraulic pump mechanism. In FIG. 2B, the pump mechanism is shown as a piston or plunger **152** that is slideable in channel **118** of introducer **110A.** In FIG. 2C, the pump mechanism is a screw pump **152'** that rotates within the channel **118** to move fill material through the channel **118..** In one embodiment, the pressure source **150** includes a controller **150B** that controls the pressure applied by the pressure source **150** (see FIG. 2A). For example, where the pressure source **150** is a piston pump or screw pump that is motor driven, the controller **150B** can adjust the motor speed to vary the pressure applied by the pressure source **150** to the inflow of the bone fill material **120.** In one embodiment, the controller **150B** also controls the volume of the bone fill material **120** that is introduced to a bone portion. In another embodiment, the controller **150B,** or a separate controller, can also control the volume of bone fill material **120** introduced into the bone portion. For example, the controller **150B** can operate a valve associated with the bone fill source **145** to selectively vary the valve opening, thus varying the volume of bone fill material **120** introduced to the bone portion.

As shown in FIGS. 2A and 2B, the introducer **110A** preferably has an electrically and thermally insulative interior sleeve **154** that defines interior flow channel **118.** The sleeve can be any suitable polymer known in the art such as PEEK, Teflon™ or a polyimide. As can be seen in FIG. 2B, interior sleeve **154** carries conductive surfaces that function as energy emitter **128,** and more particularly comprise spaced apart opposing polarity electrodes **132a** and **132b.** The electrodes **132a** and **132b** can have any spaced apart configuration and are disposed about the distal termination of channel **118** or about the surfaces of outlet **124a.** The electrode configuration alternatively can include a first electrode in the interior of channel **118** and a second electrode on an exterior of introducer **110A.** For example, the metallic sleeve **123** or a distal portion thereof can comprise one electrode. In a preferred embodiment, the electrodes **132a** and **132b** are connected to Rf energy source **125A** and controller **125B** by electrical cable **156** with (+) and (-) electrical leads **158a** and **158b** therein that extend through the insulative sleeve **154** to the opposing polarity electrodes. In one embodiment, the electrical cable **156** is detachably coupled to the handle end **106** of probe **105** by male-female plug (not shown). The electrodes **132a** and **132b** can be fabricated of any suitable materials known to those skilled in the art, such as stainless steels, nickel-titanium alloys and alloys of gold, silver platinum and the like.

In one embodiment, not shown, the working end **115A** can also carry any suitable thermocouple or temperature sensor for providing data to controller **125B** relating to the temperature of the fill material **120** during energy delivery. One or more thermocouples may be positioned at the distal tip of the introducer, or along an outer surface of the introducer and spaced from the distal end, in order to provide temperature readings at different locations within the bone. The thermocouple may also be slideable along the length of the introducer. In another embodiment, the working end can have at least one side port (not shown) in communication with a coolant source, the port configured to provide the coolant (e.g., saline) therethrough into the cancellous bone **122** to cool the cancellous bone in response to a temperature reading from the temperature sensor.

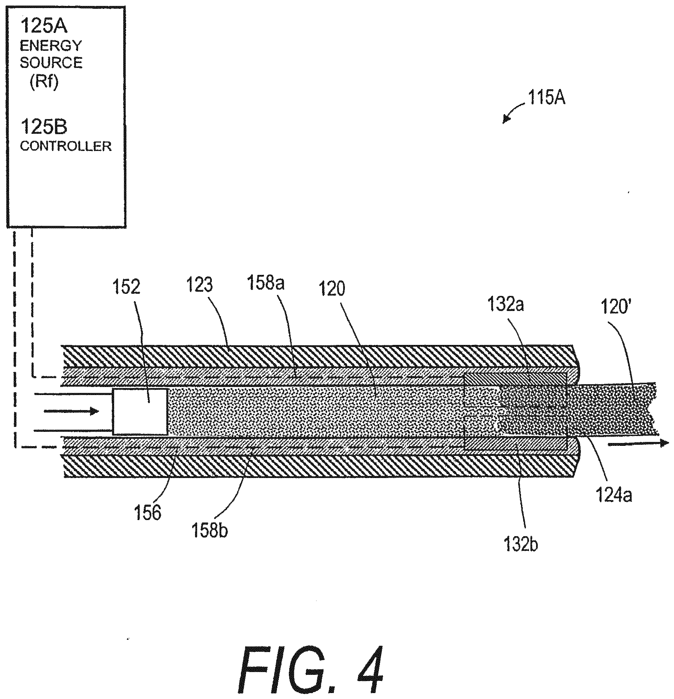

Now turning to FIG. 4, the sectional view of working end **115A** illustrates the application of energy to fill material **120** as it being ejected from outlet **124a.** The fill material **120** in the proximal portion of channel **118** can be a low viscosity flowable material such as a two-part curable polymer that has been mixed (e.g., PMMA) but without any polymerization, for example, having a viscosity of less than about 50,000 cps. Such a low viscosity fill material allows for simplified lower pressure injection through introducer 110A. Further, the system allows the use of a low viscosity fill material 120 which can save great deal of time for the physician.

Preferably it is no longer necessary to wait for the bone cement to partly polymerize before injection. As depicted in FIG. 4, energy delivery at selected parameters from electrodes 132a and 132b to fill material 120 contemporaneous with its ejection from outlet 124a selectively alters a property of fill material indicated at 120'. In one embodiment, the altered flow property is viscosity. For example, the viscosity of the fill material 120' can be increased to a higher viscosity ranging from about 100,000 cps or more, 1,000,000 cps or more, to 2,000,000 cps or more. In another embodiment, the flow property is Young's modulus. For example, the Young's modulus of the fill material 120' can be altered to be between about 10kPa and about 10GPa. In still another embodiment, the flow property can be one of durometer, hardness and compliance.

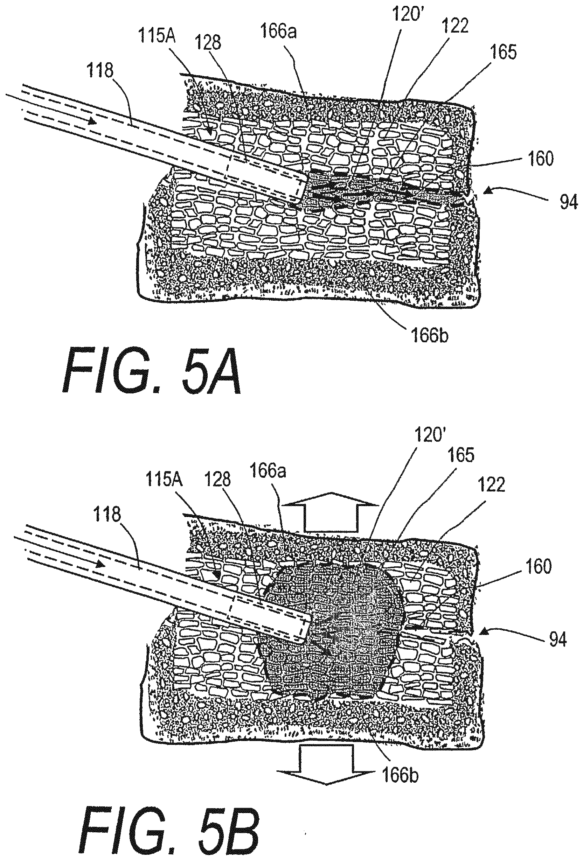

Preferably, the fill material carries a radiosensitive composition for cooperating with the Rf source 125A, as further described below. At a predetermined fill material flow rate and at selected Rf energy delivery parameters, the altered fill material 120' after ejection can comprise an increased viscosity material or an elastomer. At yet another predetermined fill material flow rate and at other Rf energy delivery parameters, the altered fill material 120' after ejection can comprise a substantially solid material or solid material. In the system embodiment utilized for vertebroplasty as depicted in FIGS. 2A and 5B, the controller is adapted for delivering Rf energy contemporaneous with the selected flow rate of fill material to provide a substantially high viscosity fill material or high viscosity fill material that is still capable of permeating cancellous bone. In other osteoplasty procedures such as treating necrosis of a bone, the system controller 125B can be adapted to provide much harder fill material 120' upon ejection from outlet 124a. Further, the system can be adapted to apply Rf energy to the fill material continuously, or in a pulse mode or in any selected intervals based on flow rate, presets, or in response to feedback from temperature sensors, impedance measurements or other suitable signals known to those skilled in the art.

In one embodiment, the controller 125B includes algorithms for adjusting power delivery applied by the energy source 125A. For example, in one embodiment the controller 125B includes algorithms for adjusting power delivery based on impedance measurements of the fill material **120'** introduced to the bone portion. In another embodiment, the controller **125B** includes algorithms for adjusting power delivery based on the volume of bone fill material **120** delivered to the bone portion. In still another embodiment, the controller **125B** includes algorithms for adjusting power delivery based on the temperature of the bone fill material **120'** introduced to the bone portion. In still another embodiment, the controller **150B** or a separate controller can include the algorithms discussed above.

FIGS. 5A and 5B are views of a vertebra **90** that are useful for explaining relevant aspects of one embodiment of the invention wherein working end **110A** is advanced into the region of fracture **94** in cancellous bone **122.** FIG. 5A indicates system **100** being used to inject flow material **120** into the vertebra with the flow material having a viscosity similar to conventional vertebroplasty or kyphoplasty, for example having the consistency of toothpaste. FIG. 5A depicts the situation wherein high pressure injection of a low viscosity material can simply follow paths of least resistance along a recent fracture plane **160** to migrate anteriorly in an uncontrolled manner. The migration of fill material could be any direction, including posteriorly toward the spinal canal or into the disc space depending on the nature of the fracture.

FIG. 5B illustrates system **100** including actuation of Rf source **125A** by controller **125B** to contemporaneously heat the fill material to eject altered fill material **120'** with a selected higher viscosity into cancellous bone **122,** such as the viscosities described above. With a selected higher viscosity, FIG. 5B depicts the ability of the system to prevent extravasion of fill material and to controllably permeate and interdigitate with cancellous bone **122,** rather than displacing cancellous bone, with a plume **165** that engages cortical bone vertebral endplates **166a** and **166b.** The fill material broadly engages surfaces of the cortical endplates to distribute pressures over the endplates. In a preferred embodiment, the fill material controllably permeates cancellous bone **122** and is ejected at a viscosity adequate to interdigitate with the cancellous bone **122.** Fill material with a viscosity in the range of about 100,000 cps to 2,000,000 cps may be ejected, though even lower or higher viscosities may also be sufficient. The Rf source may selectively increase the viscosity of the fill material by about 10% or more as it is ejected from the introducer 115A. In other embodiments, the viscosity may be increased by about 20%, 50%, 100%, 500% or 1000% or more.

Still referring to FIG. 5B, it can be understood that continued inflows of high viscosity fill material **120'** and the resultant expansion of plume **165** will apply forces on endplates **166a** and **166b** to at least partially restore vertebral height. It should be appreciated that the working end **115A** can be translated axially between about the anterior third of the vertebral body and the posterior third of the vertebral body during the injection fill material **120',** as well as rotating the working end **115A** which can be any of the types described above (FIGS. 3A-3C).

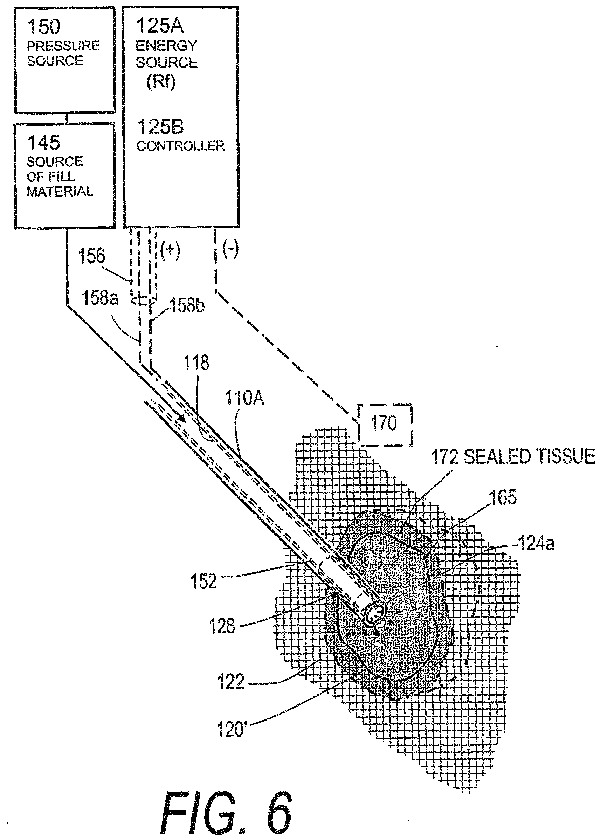

FIG. 6 is a schematic view of an alternative embodiment of system **100** wherein Rf source **125A** and controller **125B** are configured to multiplex energy delivery to provide additional functionality. In one mode of operation, the system functions as described above and depicted in FIGS. 4 and 5B to alter flow properties of flowable fill material **120'** as it is ejected from working end **115A.** As can be seen in FIG. 6, the system further includes a return electrode or ground pad indicated at **170.** Thus the system can be operated in a second mode of operation wherein electrodes **132a** and **132b** are switched to a common polarity (or the distal portion of sleeve **123** can comprise such an electrode) to function in a mono-polar manner in conjunction with ground pad **170.** This second mode of operation advantageously creates high energy densities about the surface of plume **165** to thereby ohmically heat tissue at the interface of the plume **165** and the body structure.

In FIG. 6, the ohmically heated tissue is indicated at **172,** wherein the tissue effect is coagulation of blood vessels, shrinkage of collagenous tissue and generally the sealing and ablation of bone marrow, vasculature and fat within the cancellous bone. The Rf energy levels can be set at a sufficiently high level to coagulate, seal or ablate tissue, with the controller delivering power based, for example, on impedance feedback which will vary with the surface area of plume **165.** Of particular interest, the surface of plume **165** is used as an electrode with an expanding wavefront within cancellous bone **122.** Thus, the vasculature within the vertebral body can be sealed by controlled ohmic heating at the same time that fill material **120'** is permeating the cancellous bone. Within the vertebral body are the basivertebral (intravertebral) veins which are paired valveless veins connecting with numerous venous channels within the vertebra (pars spongiosa/red bone marrow). These basivertebral veins drain directly into the external vertebral venous plexus (EVVP) and the superior and inferior vena cava. The sealing of vasculature and the basivertebral veins is particularly important since bone cement and monomer embolism has been frequently observed in vertebroplasty and kyphoplasty cases (see " Anatomical and Pathological Considerations in Percutaneous Vertebroplasty and Kyphoplasty: A Reappraisal of the Vertebral Venous System", Groen, R. et al, Spine Vol. 29, No. 13, pp 1465-1471 2004). It can be thus understood that the method of using the system 100 creates and expands a "wavefront" of coagulum that expands as the plume 165 of fill material expands. The expandable coagulum layer 172, besides sealing the tissue from emboli, contains and distributes pressures of the volume of infill material 120' about the plume surface.

The method depicted in FIG. 6 provides an effective means for sealing tissue via ohmic (Joule) heating. It has been found that passive heat transfer from the exothermic reaction of a bone cement does not adequately heat tissue to the needed depth or temperature to seal intravertebral vasculature. In use, the mode of operation of the system 100 in a mono-polar manner for ohmically heating and sealing tissue can be performed in selected intervals alone or in combination with the bi-polar mode of operation for controlling the viscosity of the injected fill material.

In general, one aspect of the vertebroplasty or osteoplasty method disclosed herein allows for in-situ control of flows of a flowable fill material, and more particularly comprises introducing a working end of an introducer sleeve into cancellous bone, ejecting a volume of flowable fill material having a selected viscosity and contemporaneously applying energy (e.g., Rf energy) to the fill material from an external source to thereby increase the viscosity of at least portion of the volume to prevent fill extravasion. In a preferred embodiment, the system increases the viscosity by about 20% or more. In another preferred embodiment, the system increases the viscosity by about 50% or more.

In another aspect of the vertebroplasty method, the system 100 provides means for ohmically heating a body structure about the surface of the expanding plume 165 of fill material to effectively seal intravertebral vasculature to prevent emboli from entering the venous system. The method further provides an expandable layer of coagulum about the infill material to contain inflow pressures and distribute further expansion forces over the vertebral endplates. Preferably the coagulum expands together with at least a portion of the infill material to engage and apply forces to endplates of the vertebra.

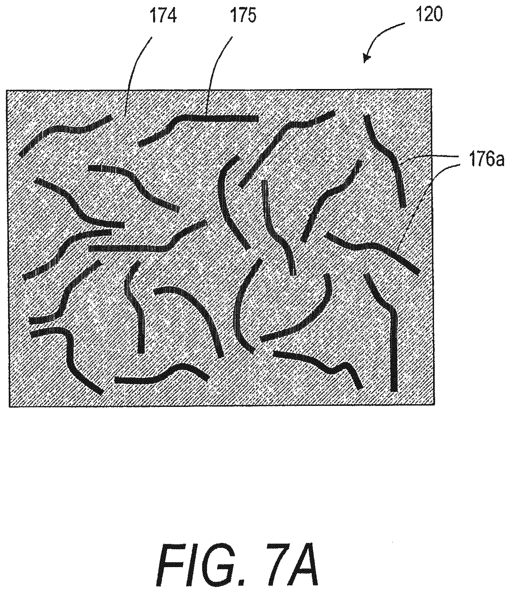

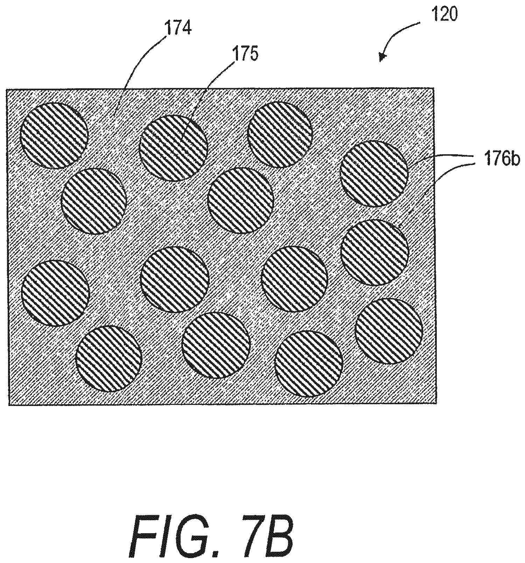

Of particular interest, one embodiment of fill material 120 as used in the systems described herein (see FIGS. 2A, 4, 5A-5B and 6) is a composite comprising an in-situ hardenable or polymerizable cement component 174 and an electrically conductive filler component 175 in a sufficient volume to enable the composite to function as a dispersable electrode (FIG. 6). In one type of composite, the conductive filler component is any biocompatible conductive metal. In another type of composite, the conductive filler component is a form of carbon. The biocompatible metal can include at least one of titanium, tantalum, stainless steel, silver, gold, platinum, nickel, tin, nickel titanium alloy, palladium, magnesium, iron, molybdenum, tungsten, zirconium, zinc, cobalt or chromium and alloys thereof. The conductive filler component has the form of at least one of filaments, particles, microspheres, spheres, powders, grains, flakes, granules, crystals, rods, tubules, nanotubes, scaffolds and the like. In one embodiment, the conductive filler includes carbon nanotubes. Such conductive filler components can be at least one of rigid, non-rigid, solid, porous or hollow, with conductive filaments 176a illustrated in FIG. 7A and conductive particles 176b depicted in FIG. 7B .

In a preferred embodiment, the conductive filler comprises chopped microfilaments or ribbons of a metal as in FIG. 7A that have a diameter or a cross-section dimension across a major axis ranging between about 0.0005" and 0.01". The lengths of the microfilaments or ribbons range from about 0.01" to 0.50". The microfilaments or ribbons are of stainless steel or titanium and are optionally coated with a thin gold layer or silver layer that can be deposited by electroless plating methods. Of particular interest, the fill material 120 of FIG. 7A has an in situ hardenable cement component 174 than has a first low viscosity and the addition of the elongated microfilament conductive filler component 175 causes the composite 120 to have a substantially high or high apparent viscosity due to the high surface area of the microfilaments and its interaction with the cement component 174. In one embodiment, the microfilaments are made of stainless steel, plated with gold, and have a diameter of about 12 microns and a length of about 6 mm. The other dimensions provided above and below may also be utilized for these microfilaments.

In another bone fill material 120, the conductive filler component comprises elements that have a non-conductive core portion with a conductive cladding portion for providing electrosurgical functionality. The non-conductive core portions are selected from the group consisting of glass, ceramic or polymer materials.
The cladding can be any suitable conductive metal as described above that can be deposited by electroless plating methods.

In any bone fill material that uses particles, microspheres, spheres, powders, grains, flakes, granules, crystals or the like, such elements can have a mean dimension across a principal axis ranging from about 0.5 micron to 2000 microns. More preferably, the mean dimension across a principal axis range from about 50 microns to 1000 microns. It has been found that metal microspheres having a diameter of about 800 microns are useful for creating conductive bone cement that can function as an electrode.

In one embodiment, a conductive filler comprising elongated microfilaments wherein the fill material has from about 0.5% to 20% microfilaments by weight. More preferably, the filaments are from about 1% to 10% by weight of the fill material. In other embodiments wherein the conductive filler comprises particles or spheres, the conductive filler can comprise from about 5% of the total weight to about 80% of the weight of the material.

In another fill material 120, the hardenable component can be any in-situ hardenable composition such as at least one of PMMA, monocalcium phosphate, tricalcium phosphate, calcium carbonate, calcium sulphate or hydroxyapatite.

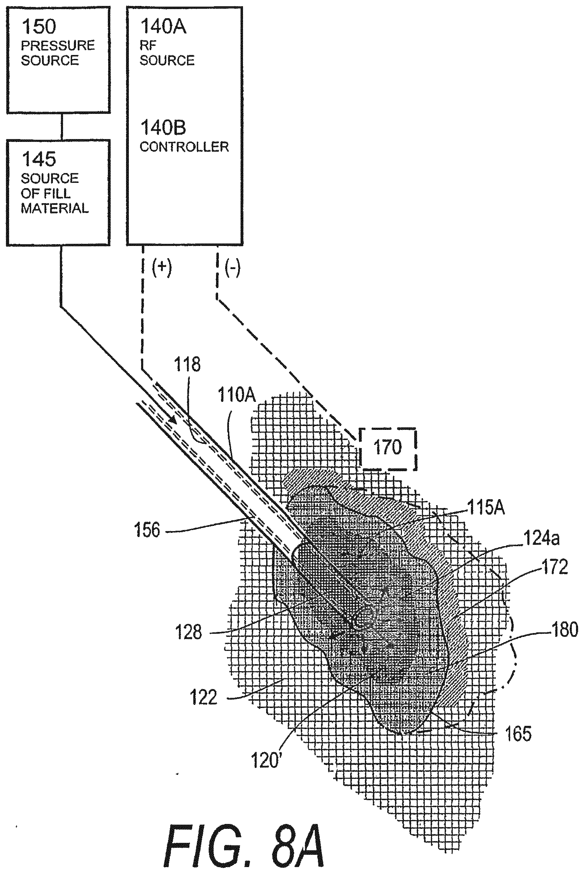

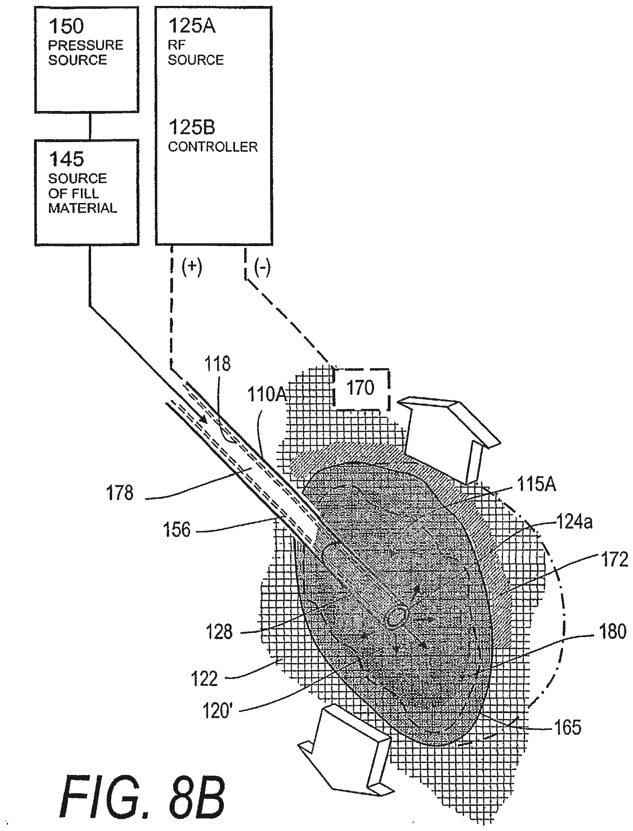

Referring now to FIGS. 8A and 8B , an alternative method is shown wherein the system 100 and method are configured for creating asymmetries in properties of the infill material and thereby in the application of forces in a vertebroplasty. In FIG. 8A , the pressure mechanism 150 is actuated to cause injection of an initial volume or aliquot of fill material 120' that typically is altered in viscosity in working end 110A as described above-but the method encompasses flows of fill material having any suitable viscosity. The fill material is depicted in FIGS. 8A and 8B as being delivered in a unilateral transpedicular approach, but any extrapedicular posterior approach is possible as well as any bilateral posterior approach. The system in FIGS. 8A-8B again illustrates a vertical plane through the fill material 120' that flows under pressure into cancellous bone 122 with expanding plume or periphery indicated at 165. The plume 165 has a three dimensional configuration as can be seen in FIG. 8B, wherein the pressurized flow may first tend to flow more horizontally than vertically. One method includes the physician translating the working end slightly and/or rotating the working end so that flow outlets 124a are provided in a selected radial orientation. Preferably the physician intermittently monitors the flows under fluoroscopic imaging as described above.

FIG. 8B depicts a contemporaneous or subsequent energy-delivery step of the method wherein the physician actuates Rf electrical source 125A and controller 125B to cause Rf current delivery from at least one electrode emitter 128 to cause ohmic (Joule) heating of tissue as well as internal heating of the inflowing fill material 120'. The exterior surface of sleeve 123 is indicated as electrode or emitter 128 with the proximal portion of introducer 110A having an insulator coating 178. In another embodiment, the proximal portion of the introducer 110A has an area of non-conductive plastic. The Rf energy is preferably applied in an amount and for a duration that coagulates tissue as well as alters a flowability property of surface portions 180 of the initial volume of fill material proximate the highest energy densities in tissue.

The fill material 120 is particularly designed to create *gradient* in the distribution of conductive filler with an increase in volume of material injected under high pressure into cancellous bone 122. This aspect of the method in turn can be used advantageously to create asymmetric internal heating of the fill volume. The fill material 120 may include a conductive filler of elongated conductive microfilaments 176a (FIG. 7A). The filaments are from about 2% to 5% by weight of the fill material, with the filaments having a mean diameter or mean sectional dimension across a minor axis ranging between about 0.001" and 0.010" and a length ranging from about 1 mm to about 10 mm, more preferably about 1 mm to 5 mm. In another embodiment, the filaments have a mean diameter or a mean dimension across a minor axis ranging between about 1 micron and 500 microns, more preferably between about 1 micron and 50 microns, even more preferably between about 1 micron and 20 microns. It has been found that elongated conductive microfilaments 176a result in resistance to flows thereabout which causes such microfilaments to aggregate away from the most active media flows that are concentrated in the center of the vertebra proximate to outlet 124a. Thus, the conductive microfilaments 176a attain a higher concentration in the peripheral or surface portion 180 of the plume which in turn will result in greater internal heating of the fill portions having such higher concentrations of conductive filaments. The active flows also are controlled by rotation of introducer 110A to eject the material preferentially, for example laterally as depicted in FIG. 8A and 8B rather that vertically. The handle 106 of the probe 105 preferably has markings to indicate the rotational orientation of the outlets 124b.

FIG. 8A depicts the application of Rf energy in a monopolar manner between electrode emitter 128 and ground pad 170, which thus causes asymmetric heating wherein surface portion 180 heating results in greater polymerization therein. As can be seen in FIG. 8A, the volume of fill material thus exhibits a gradient in a flowability property, for example with surface region 180 having a higher viscosity than inflowing material 120' as it is ejected from outlet 124a. The gradient may be continuous. Such heating at the plume periphery 165 can create an altered, highly viscous surface region 180. This step of the method can transform the fill material to have a gradient in flowability in an interval of about 5 seconds to 500 seconds with surface portion 180 being either a highly viscous, flowable layer or an elastomer that is expandable. The interval of energy delivery required is preferably less than about 120 seconds to alter fill material to a selected asymmetric condition. The Rf energy application for creating the gradient in flowability also can be optimized for coagulating and sealing adjacent tissue.

The combination of the viscous surface portion 180 and the tissue coagulum 172 may function as an in-situ created stretchable, but substantially flow-impervious or flow-impervious, layer to contain subsequent high pressure inflows of fill material. Thus, the next step of the method is depicted in FIG. 8B which includes injecting additional fill material 120' under high pressure into the interior of the initial volume of fill material 120 that then has a highly viscous, expandable surface. The viscous, expandable surface desirably surrounds cancellous bone. By this means, the subsequent injection of fill material can expand the fill volume to apply retraction forces on the vertebra endplates 166a and 166b to provide vertical jacking forces, distracting cortical bone, for restoring vertebral height, as indicated by the arrows in FIG. 8B. The system can generate forces capable of breaking callus in cortical bone about a vertebral compression fracture when the fracture is less than completely healed.

The method includes applying Rf energy to create highly viscous regions in a volume of fill material and thereafter injecting additional fill material 120 to controllably expand the fill volume and control the direction of force application. The method further includes applying Rf energy in multiple intervals or contemporaneous with a continuous flow of fill material. The method also includes applying Rf in conjunction with imaging means to prevent unwanted flows of the fill material. The method may also include applying Rf energy to polymerize and accelerate hardening of the entire fill volume after the desired amount of fill material has been injected into a bone.

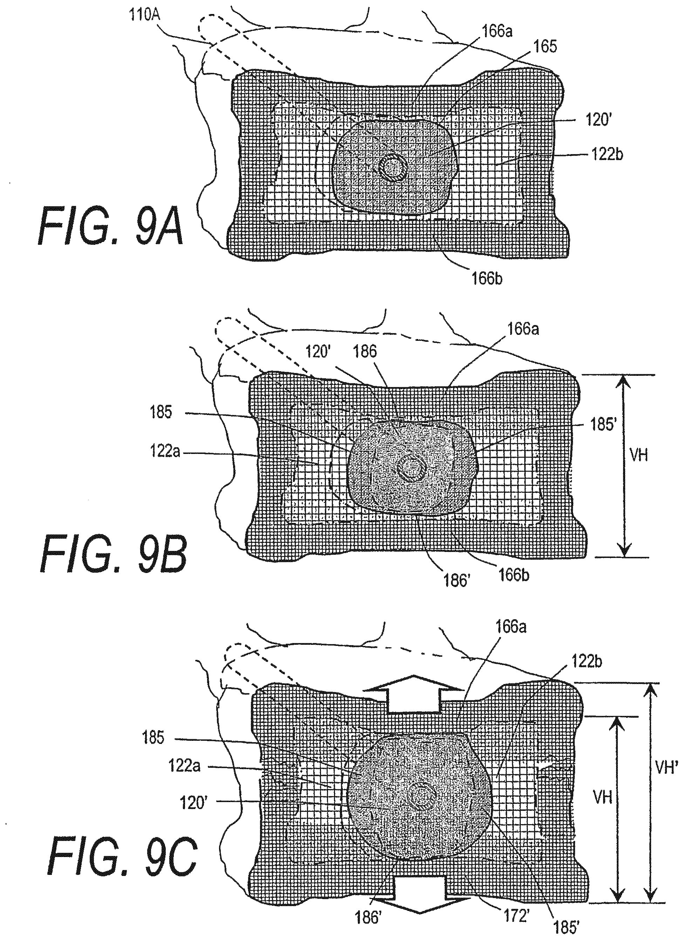

The method may include creating Rf current densities in selected portions of the volume of fill material 120 to create asymmetric fill properties based on particular characteristics of the vertebral body. For example, the impedance variances in cancellous bone and cortical bone can be used to create varied Rf energy densities in fill material 120 to create asymmetric properties therein. Continued injection of fill material 120 are thus induced to apply asymmetric retraction forces against cortical endplates 166a and 166b, wherein the flow direction is toward movement or deformation of the lower viscosity portions and away from the higher viscosity portions. In FIGS. 9A-9C, it can be seen that in a vertebroplasty, the application of Rf energy in a mono-polar manner as in FIG. 6 naturally and preferentially creates more highly viscous, deeper "altered" properties in surfaces of the lateral peripheral fill volumes indicated at 185 and 185' and less viscous, thinner altered surfaces in the superior and inferior regions 186 and 186' of fill material 120. This effect occurs since Rf current density is localized about paths of least resistance which are predominantly in locations proximate to highly conductive cancellous bone 122a and 122b. The Rf current density is less in locations proximate to less conductive cortical bone indicated at 166a and 166b. Thus, it can be seen in FIG. 9B that the lateral peripheral portions 185 and 185' of the first flows of fill material 120 are more viscous and resistant to flow and expansion than the thinner superior and inferior regions. In FIG. 9C , the asymmetrical properties of the initial flows of fill material 120 allows the continued flows to apply retraction forces in substantially vertical directions or vertical directions to reduce the vertebral fracture and increase vertebral height, for example from VH (FIG. 9B) to VH' in FIG. 9C .

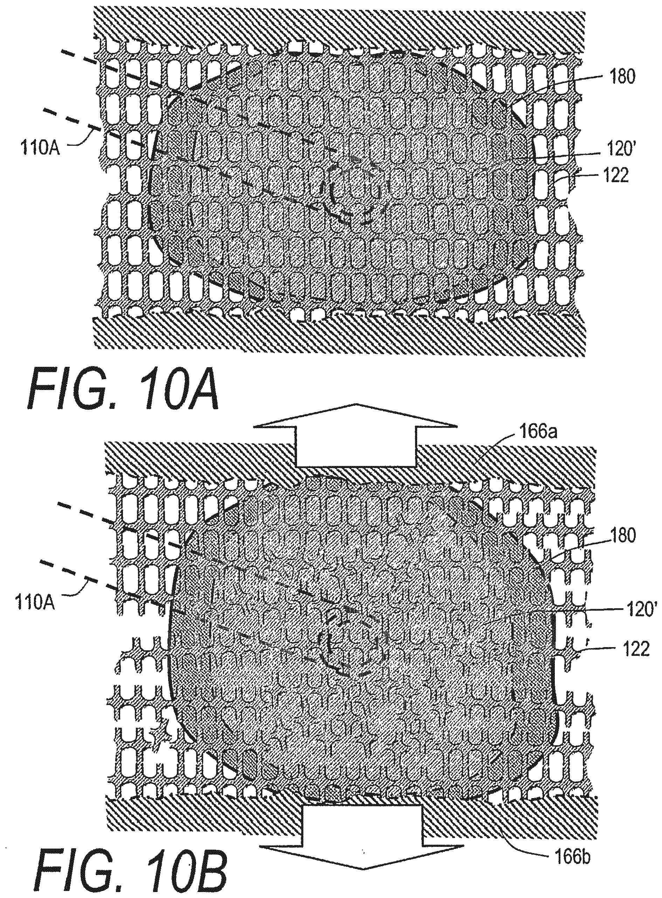

FIGS. 10A and 10B are schematic views that further depict a method corresponding to FIGS. 9B and 9C that comprises expanding cancellous bone for applying retraction forces against cortical bone, e.g., endplates of a vertebra in a vertebroplasty. As can be seen in FIG. 10A, an initial volume of flowable fill material 120 is injected into cancellous bone wherein surface region 180 is altered as described above to be highly viscous or to comprise and elastomer that is substantially impermeable or impermeable to interior flows but still be expandable. The surface region 180 surrounds subsequent flows of fill material 120' which interdigitate with cancellous bone. Thereafter, as shown in FIG. 10B, continued high pressure inflow into the interior of the fill material thereby expands the cancellous bone 122 together with the interdigitated fill material 120'. As can be seen in FIG. 10B, the expansion of cancellous bone 122 and fill material 120' thus applies retraction forces to move cortical bone endplates 166a and 166b. The method of expanding cancellous bone can be used to reduce a bone fracture such as a vertebral compression fracture and can augment or restore the height of a fractured vertebra. The system thus can be used to support retract and support cortical bone, and cancellous bone. The method can also restore the shape of an abnormal vertebra, such as one damaged by a tumor. In the event intraoperative imaging shows fill material flowing in an undesirable direction or vector, Rf energy can be applied to polymerize the fill material, which will affect the surface more greatly than the interior of the volume of fill material. Thereafter, the physician can translate and/or rotate the introducer 110A to direct the outlets 124b to create distraction forces in the desired direction. These steps can be repeated a number of times to reduce a vertebral fracture and control the direction of material flow.

After utilizing system 100 to introduce, alter and optionally harden fill material 120 as depicted in FIGS. 9A-9C and 10A-10B, the introducer 110A can be withdrawn from the bone. Alternatively, the introducer 110A can have a release or detachment structure indicated at 190 for de-mating the working end from the proximal introducer portion. The release or detachment structure can be any suitable mechanism, such as a screw thread, a releasable clamp, a thermally sacrificial polymer, a fracturable element, or a scored frangible structure that is broken by extension forces. In another embodiment, the detachment structure can include the system invented for spacecraft, which can be adapted for medical use. In such an embodiment, the system is a nickel titanium (NiTi) actuated frangibolt system, as known in the art, which was developed to replace explosive bolts in satellite deployment. The system would include a resistively heated NiTi actuator to separate the implantable medical device working end from the introducer 110A or catheter based on frangibolt designs disclosed in U.S. Patent No. 5,119,555, and commercialized by TiNi Aerospace, Inc. of San Leandro, California. The system thus de-couples the proximal portion of the introducer 110A from the distal portion thereof, leaving the working end in the vertebra. Further details on a release or detachment structure can be found in U.S. Patent Application No. 11/130,843, filed May 16, 2005.

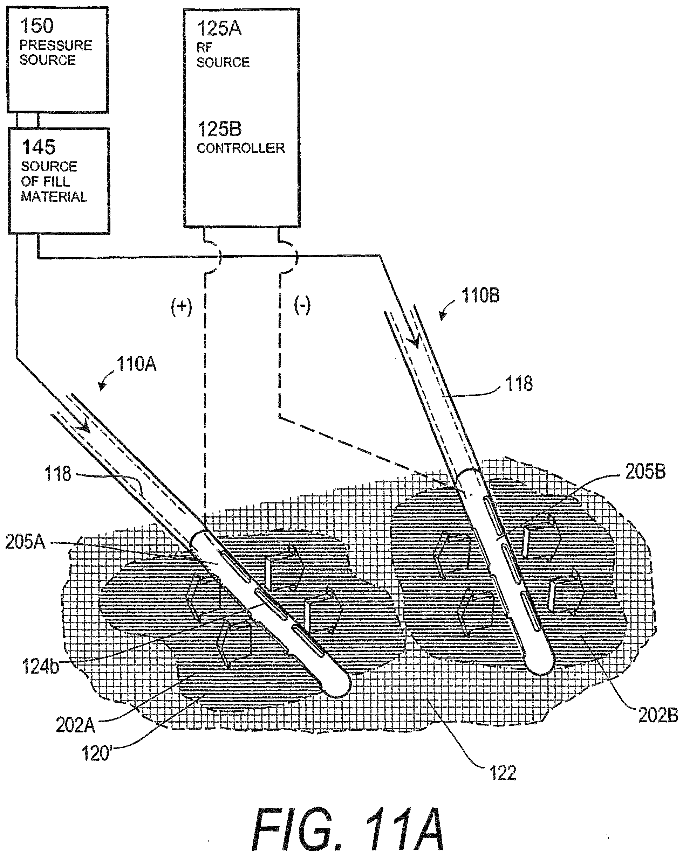

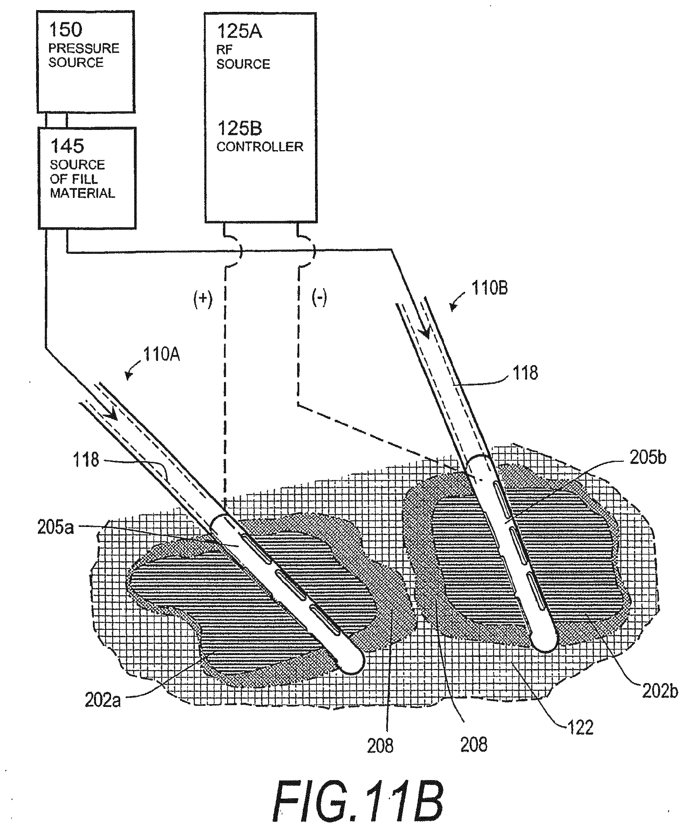

Another system embodiment 200 for controlling flow directions and for creating asymmetric properties is shown in FIGS. 11A and 11B , wherein first and second introducers 110A and 110B similar to those described above are used to introduce first and second independent volumes 202a and 202b of fill material 120 in a bilateral approach. In this embodiment, the two fill volumes function as opposing polarity electrodes in contact with electrodes 205a and 205b of the working ends. Current flow between the electrodes thus operates in a bi-polar manner with the positive and negative polarities indicated by the (+) and (-) symbols. In this method, it also can be seen that the highest current density occurs in the three dimensional surfaces of volumes 202a and 202b that face one another. This results in creating the thickest, high viscosity surfaces 208 in the medial, anterior and posterior regions and the least "altered" surfaces in the laterally outward regions. This method is well suited for preventing posterior and anterior flows and directing retraction forces superiorly and inferiorly since lateral flow are contained by the cortical bone at lateral aspects of the vertebra. The system can further be adapted to switch ohmic heating effects between the bi-polar manner and the mono-polar manner described previously.

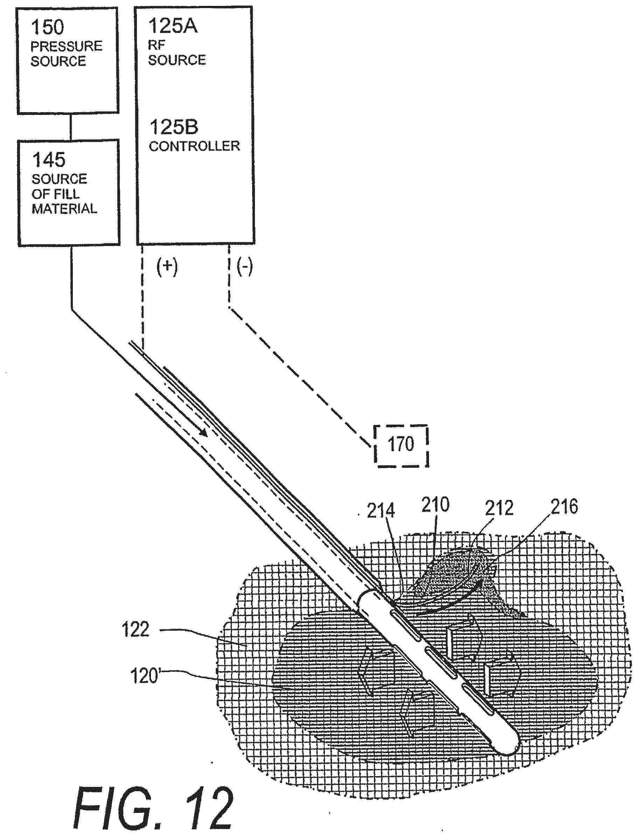

Now referring to FIG. 12, another embodiment is shown wherein a translatable member 210 that functions as an electrode is carried by introducer 110A. In a preferred embodiment, the member 210 is a superelastic nickel titanium shape memory wire that has a curved memory shape. The member 210 can have a bare electrode tip 212 with a radiopaque marking and is otherwise covered by a thin insulator coating. In FIG. 12, it can be seen that the introducer can be rotated and the member can be advanced from a port 214 in the working end 115A under imaging. By moving the electrode tip 212 to a desired location and then actuating RF current, it is possible to create a local viscous or hardened region 216 of fill material 120. For example, if imaging indicates that fill material 120 is flowing in an undesired direction, then injection can be stopped and Rf energy can be applied to harden the selected location.

In another embodiment similar to the one shown in FIG. 12, the translatable member 210 can comprise a hollow needle that injects a chemical agent (e.g., a catalyst) to accelerate local curing of the fill material 120. Alternatively, the hollow needle can deliver a microencapsulated chemical agent that is released by Rf energy delivery to sacrifice the microcapsule.

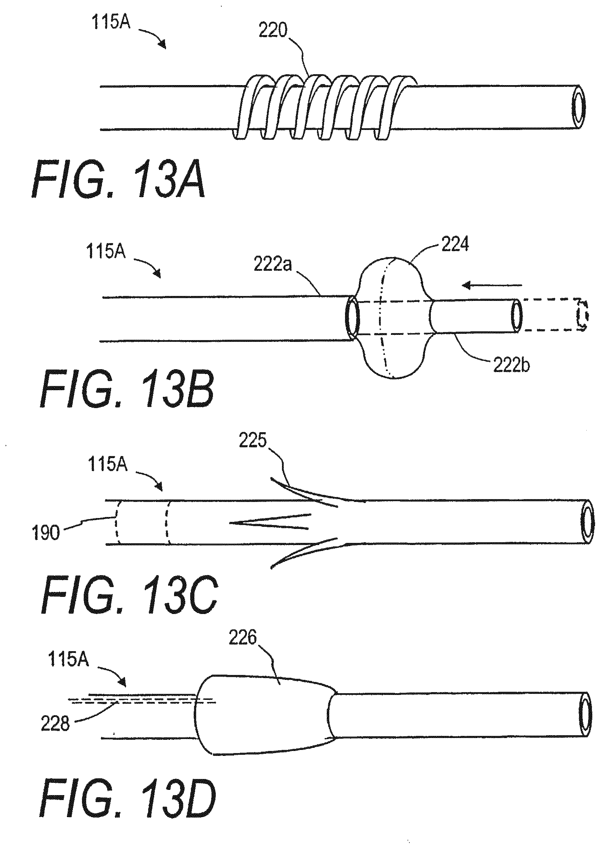

FIGS. 13A-13D illustrate other versions of the introducer 110A, which include structures for engaging the working end 115A in bone to substantially prevent or prevent it from moving proximally when very high pressures are used to inject bone fill material 120, for example to augment vertebral height when treating a VCF. FIG. 13A illustrates a working end with threads 220 for helically advancing the introducer which will secure the introducer in bone. FIG. 13B illustrates a working end with first and second concentric sleeves 222a and 222b that can be used to buckle and radially expand a resilient element 224 such as a rubber member. Alternatively, the system of FIG. 13B could be configured to buckle at least one metal element. FIG. 13C illustrates a working end with barbs 225 that engage the bone as the structure is moved proximally. In the illustrated embodiment, such a working end can be detached using a detachment mechanism indicated at 190 as described above. In another embodiment, the introducer barbs 225 can be configured to collapse somewhat under rotation to thereby rotate and withdraw the introducer from bone. FIG. 13D illustrates a working end with an expandable balloon structure 226 for gripping bone that is inflated through lumen 228 from an inflation source.

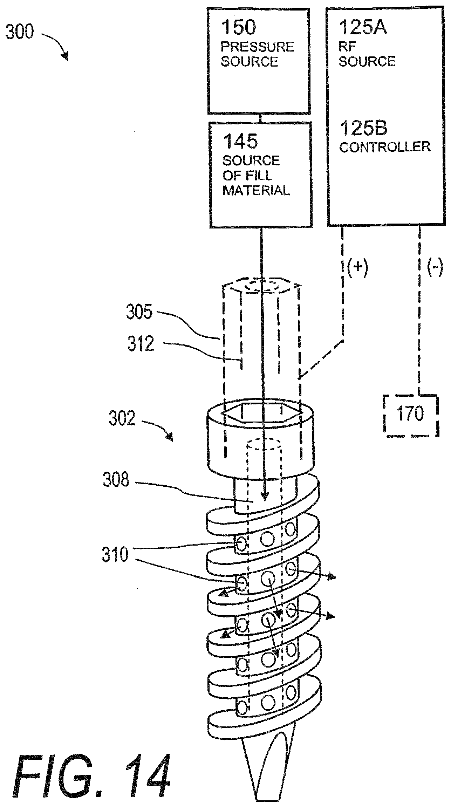

FIG. 14 illustrates another embodiment of the invention wherein the on-demand hardenable fill material 120 is combined with an implant 300 such as a bone screw, pin, shaft, joint reconstruction body or the like. As one example of an implant, FIG. 14 illustrates a metal bone screw 302 that cooperates with driver member 305 for helically driving the screw. The bone screw 302 has a lumen 308 that communicates with a plurality of outlets 310 in the implant body. In one embodiment, the driver 305 has a cooperating bore 312 that is coupled to a source 145 of conductive fill material 120 as described above. Further, the system includes Rf source 125A and controller 125B for applying Rf energy to harden the fill material on demand. In one embodiment, the Rf source is coupled to the electrically conductive driver 305 which carries Rf current to the bone screw by contact. As can be seen in FIG. 14, the method includes driving the bone screw in a bone, and then injecting the fill material 120 which will flow through outlets 310 (see arrows) in the implant. Thereafter, the Rf source is actuated to cure the fill material 120 to thereby fix the implant in bone.

It should be appreciated that the system FIG. 14 can be coupled with any type of bone implant, including joint reconstruction components for hips, knees, shoulders and the like, ligament or tendon implants that are fixed in a bore in bone, reconstructive surgery implants, and any other screw, pin or plate or the like.

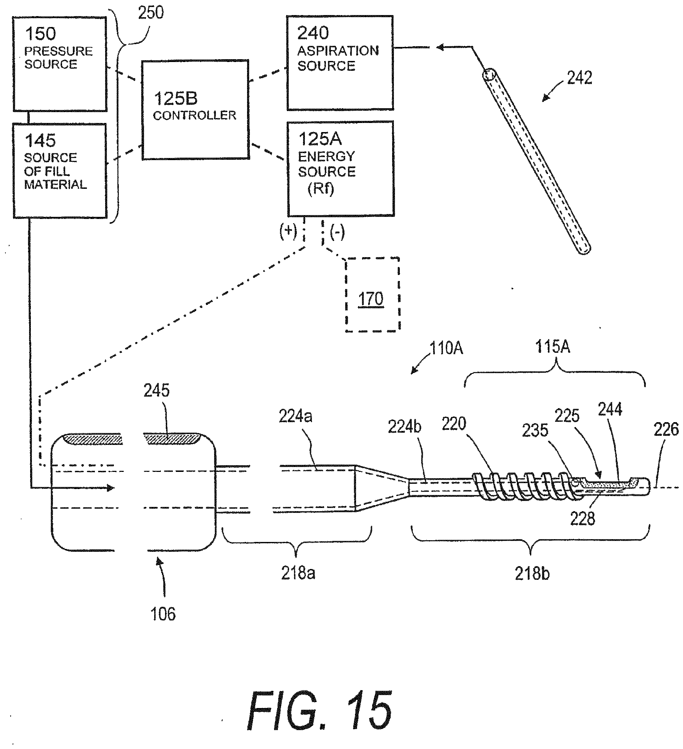

FIG. 15 illustrates another introducer 110A which includes a transition in cross-sectional dimension to allow for decreased pressure requirements for introducing bone cement through the length of the introducer. In FIG. 15, the proximal handle end 106 is coupled to introducer 110A that has a larger diameter proximal end portion 218a that transitions to a smaller diameter distal end portion 218b configured for insertion into a vertebral body. The distal end portion 218b includes exterior threads 220 for helical advancement and engagement in bone to prevent the introducer from moving proximally when cement is injected into a vertebral body or other bone, for example to augment vertebral height when treating a VCF. The bore that extends through the introducer 110A similarly transitions from larger diameter bore portion 224a to smaller diameter bore portion 224b. The embodiment of FIG. 15 utilizes a bore termination or slot 225 in a sidewall of the working end 115A for ejecting bone cement at a selected radial angle from the axis 226 of the introducer for directing cement outflows within a vertebral body.

Still referring to FIG. 15, the introducer 110A is coupled to bone cement source 145 and pressure source 150 as described previously that is controlled by controller 125B. Further, an energy source 125A (e.g., Rf source) is coupled to an energy delivery mechanism in the working end 115A for applying energy to a cement flow within bore 224b. In the embodiment of FIG. 15 , the introducer can be fabricated of a strong reinforced plastic such a polymide composite with a sleeve electrode 228 in bore 224a and inward of the bore termination slot 225, similar to electrode 128 depicted in FIG. 3A . The electrode 228 in FIG. 15 is coupled to Rf source 125A for operating in a mono-polar manner in cooperation with a return ground pad indicated at 170. The controller 125B again is operatively connected to Rf source 125A to adjust energy delivery parameters in response to feedback from a thermocouple 235 in the bore 124a or in response to measuring impedance of the cement flow. In FIG. 15, the controller 125B further is operationally connected to an aspiration source 240 that is coupled to a needle-like introducer sleeve 242 that can be inserted into a bone to apply suction forces to the interior of vertebra for relieving pressure in the vertebra and/or extracting fluids, bone marrow and the like that could migrate into the venous system. The use of such an aspiration system will be described further below.

In FIG. 15, the introducer 110A has a larger diameter bore 224a that ranges from about 4 mm. to 10 mm. and preferably is in the range of about 5 mm to 6 mm. The smaller diameter bore 224b can range from about 1 mm. to 3 mm. and preferably is in the range of about 1.5 mm to 2.5 mm. The exterior threads 220 can be any suitable height with single or dual flights configured for gripping cancellous bone. The thread height and length of the reduced diameter section 218b are configured for insertion into a vertebra so that the port 225 can be anteriorly or centrally located in the vertebral body. The working end 115A further carries a radiopaque marking 244 for orienting the radial angle of the introducer and bore termination port 225. In FIG. 15, the radiopaque marking 244 is elongated and surrounds port 225 in the introducer sidewall. The handle 106 also carries a marking 245 for indicating the radial angle of port 225 to allow the physician to orient the port by observation of the handle.

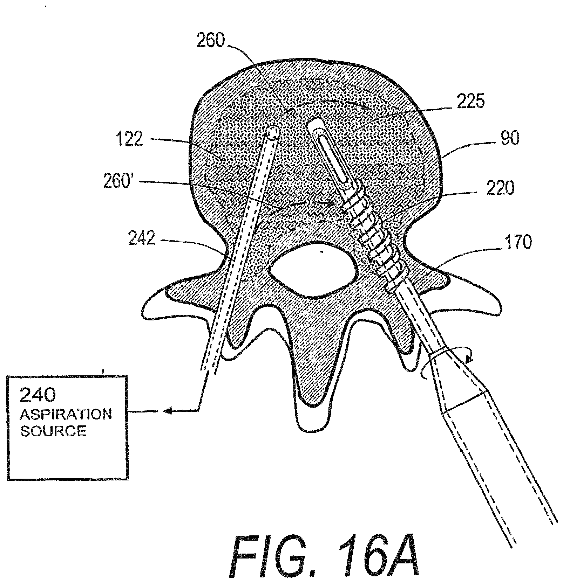

Now referring to FIGS. 16A-16C, the working end 115A of the introducer of FIG. 15 is shown after being introduced into cancellous bone 122 in vertebra 90. FIG. 16A illustrates a horizontal sectional view of vertebra 90 wherein the bore termination port 225 is oriented superiorly to direct cement inflows to apply forces against cancellous bone 122 and the superior cortical endplate 248 of the vertebra. A method comprises providing a flow source 250 (the pressure source 150 and cement source 145, in combination, are identified as flow source 250 in FIG. 15) for bone cement inflows and a controller 125B for control of the bone cement inflows, and inflowing the bone cement into a vertebral body wherein the controller adjusts an inflow parameter in response to a measured characteristic of the cement. In one method, the measured characteristic is temperature of the bone cement measured by thermocouple 235 in the working end 115A. The controller 125B can be any custom computerized controller. The system can utilize a commercially available controller manufactured by EFD Inc., East Providence, RI 02914, USA for flow control, wherein either a positive displacement dispensing system or an air-powered dispensing system can coupled to the flow source 250. In response to feedback from thermocouple 235 that is read by the controller 125B, any inflow parameter of the bone cement flow can be adjusted, for example cement injection pressure, the inflow rate or velocity of the bone cement flows or the acceleration of a bone cement flow. The controller 125B also can vary any inflow parameter with time, for example, in pulsing cement inflows to thereby reduce a vertebral fracture, or move cancellous or cortical bone (see FIGS. 16A-16B). The cement 120 can be introduced in suitable volumes and geometries to treat fractures or to prophylactically treat a vertebra.

In another method, the flow source 250, controller 125B and Rf energy source 125A are provided as shown in FIG. 15. The controller 125B again is capable of adjusting any bone cement delivery parameter in response to impedance and/or temperature. The controller 125B adjusts at least one cement delivery parameter selected from cement volume, pressure, velocity and acceleration of the inflowing cement. The controller 125B also can vary pressure of the inflowing cement or pulse the cement inflows. The controller 125B also is capable of adjusting energy delivered from Rf energy source 125A to the inflowing cement 120 in response to impedance, temperature, cement viscosity feedback or cement flow parameters to alter cement viscosity as described above. Cement viscosity can be calculated by the controller 125B from temperature and pressure signals. The controller 125B also is capable of being programmed with algorithms to ramp-up and ramp down power in one or more steps, or can be programmed to pulse power delivery to the bone cement 120 (FIGS. 16A-16B).

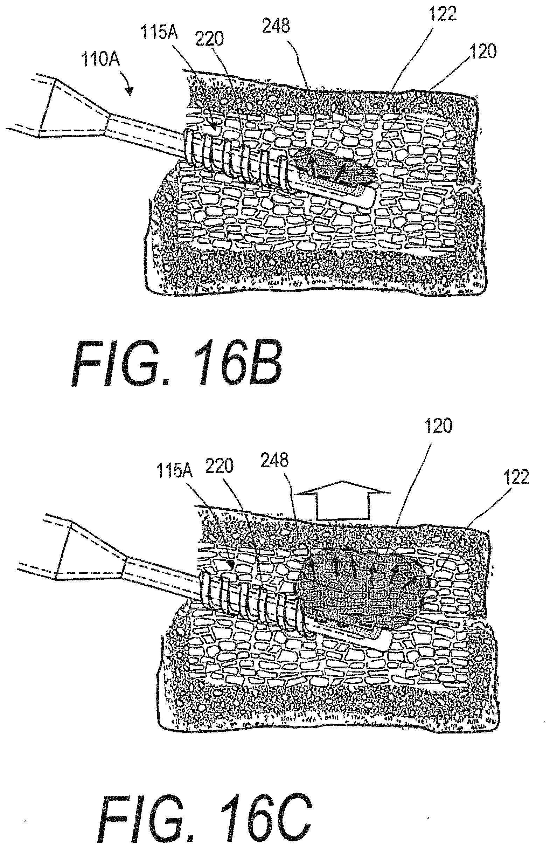

As can be seen in FIGS. 16B and 16C, the inflowing cement 120 can be directed to apply forces against cancellous bone 122 and the superior cortical endplate 248 of the vertebra, or the working end can be rotated to introduce cement 120 and apply forces in other directions. The extension of the working end 115A in cancellous bone serves as a support for causing expansion pressures to be directed in the direction of cement flows. The method of treating the vertebra includes translating (by helical advancement) and rotating the introducer 110A to thereby alter the direction of cement introduction. In another embodiment (not shown), the introducer 110A can comprise an assembly of first and second concentric sleeves wherein the outer sleeve has threads 220 for locking the assembly in bone and the inner sleeve is rotatable to adjust the angular direction of port 225 wherein the sleeves are locked together axially. This embodiment can be used to intermittently angularly adjust the direction of cement outflows while helical movement of the outer sleeve adjusts the axial location of port 225 and the cement outflows.

In another method, referring back to FIG. 16A, the aspiration introducer sleeve 242 can be inserted into the vertebral body, for example through the opposing pedicle. The controller 125B can be programmed to alter aspiration parameters in coordination with any bone cement inflow parameter. For example, the cement inflows can be pulsed and the aspiration forces can be pulsed cooperatively to extract fluids and potentially embolic materials, with the pulses synchronized. In one method, the cement inflows are pulsed at frequency ranging between about 1 per second and 500 per second with an intense, high acceleration pulse which causes bone marrow, fat, blood and similar materials to become susceptible to movement while at the same time the aspiration pulses are strong to extract some mobile marrow etc into the aspiration sleeve 242. In FIG. 16A, the aspiration sleeve 242 is shown with single port in it distal end. It should be appreciated that the scope of the invention and its method of use includes an aspiration sleeve 242 that has a plurality of inflow ports along the length of the sleeve and the sleeve also can be curved or can be of a shape memory alloy (e.g., Nitinol) to for introduction in a curved path in the anterior of posterior region of a vertebral body as indicated by lines 260 and 260' in FIG. 16A. In another embodiment, the aspiration sleeve 242 can extend through the introducer 110A or can comprise an outer concentric sleeve around the introducer 110A.

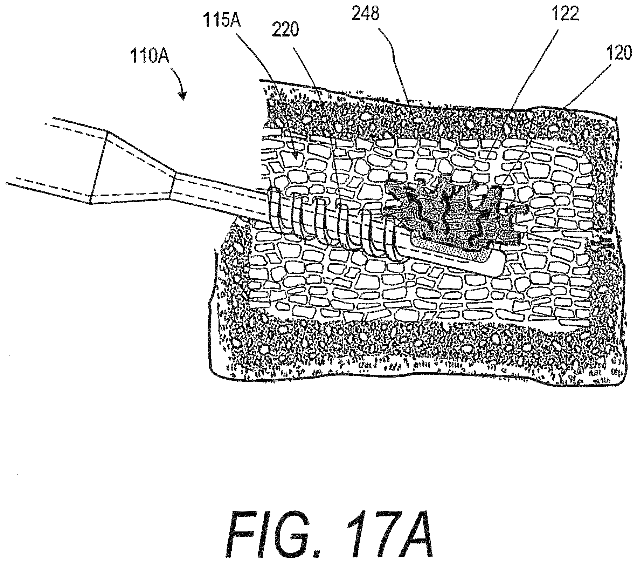

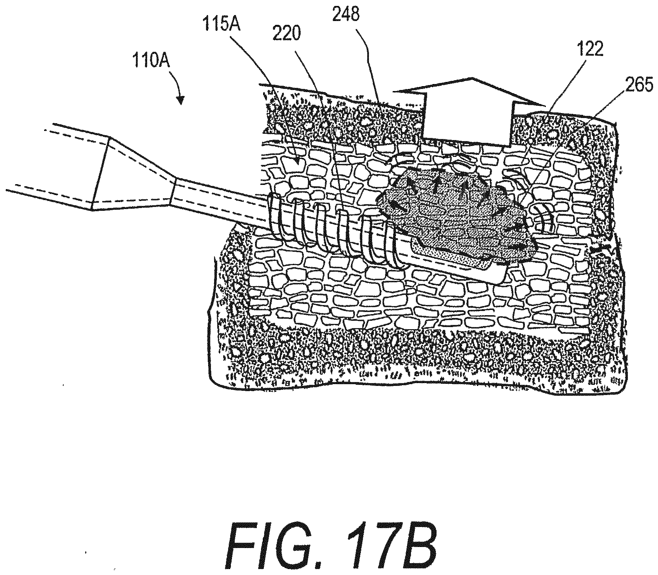

FIGS. 17A and 17B illustrates another method wherein the controller 125B and pressure source 150 are configured to introduce a flowable cement into the interior of a vertebra one under widely varying velocities and rates of acceleration to optionally (i) provide first slow flow rates to allow cement flow and interdigitation into and through cancellous bone, and (ii) provide second higher flow rates that disallow cement interdigitation and flow into and through cancellous bone. At suitable high acceleration and flow velocity, for example in a pulse of cement flow into bone, the accelerated flow apply forces to bone across the surface of the cement plume which can displace cancellous bone rather than allowing the cement to flow into the cancellous bone. FIG. 16A illustrates the system of FIG. 15 in a method of use wherein the controller 125B and pressure source 150 are actuated to cause a volume of cement 120 to flow into cancellous bone 122 under a suitable low pressure to allow the cement to interdigitate with, and flow into, the cancellous bone. The flow of cement depicted in FIG. 16A can be accompanied by the application of aspiration forces as described above. FIG. 17B illustrates another aspect of the method wherein the controller 125B and pressure source 150 are actuated to flow cement with a high acceleration rate and velocity that disallows the cement from having time to flow into pores of the cancellous bone. The acceleration and velocity are selected to disallow cement interdigitation which thereby causes the application of force to bone across the surface of the cement plume 265 (FIG. 17B). The application of such forces across the surface of cement plume 265 is further enabled by providing a suitable high viscosity cement as described above, which includes selectively increasing cement viscosity by means of energy delivery thereto. The method can include one or more sequences of flowing cement into the bone to first cause cement interdigitation (FIG. 17A) and then to apply expansion forces to the bone by at least one high acceleration flow (FIG. 17B). Of particular interest, the method of using high acceleration flows, for example in pulses, causes the cement volume to apply forces to bone akin to the manner is which a mechanical expander or balloon expander would apply forces to bone. That is, expansion forces are applied across the entire surface of cement plume 265 similar to then manner in which mechanical instruments apply expanding forces across the bone engaging surface of the instrument. The methods are adapted for reducing a vertebral fracture and for selectively applying forces to move cancellous bone and cortical bone.

Also disclosed is cure-on-demand fill material that can be used for disc nucleus implants, wherein the conductive fill material in injected to conform to the shape of a space wherein Rf current is then applied to increase the modulus of the material on demand to a desired level that is adapted for dynamic stabilization. Thus, the Rf conductive filler material 120 can be engineered to reach a desired modulus that is less than that of a hardened fill material used for bone support. The fill material is used to support a disc or portion thereof. The cure-on-demand fill material also can be configured as and injectable material to repair or patch a disc annulus as when a tear or herniation occurs.

Also disclosed is cure-on-demand fill material that can be used for injection into a space between vertebrae for intervertebral fusion. The injection of fill material can conform to a space created between two adjacent vertebrae, or can be injected into notches or bores in two adjacent vertebrae and the intervening space, and then cured by application of Rf current to provide a substantially high modulus block or high modulus block to cause bone fusion.

In any embodiment such as for intervertebral fusion or for bone support in VCFs, the system can further include the injection of a gas (such as carbon dioxide) into the fill material before it is injected from a high pressure source. Thereafter, the gas can expand to form voids in the fill material as it is cured to create porosities in the hardened fill material for allowing rapid bone ingrowth into the fill material.

In a related method, the fill material 120 can be introduced into the cancellous bone 122 in different aliquots wherein each volume carries a different type of conductive filler, e.g., with different volume percentages of conductive filler or different dimensions of conductive fillers. In one embodiment, the secondary aliquots of fill material are not conductive.

In related methods, the system of the invention can use any suitable energy source, other that radiofrequency energy, to accomplish the purpose of altering the viscosity of the fill material 120. The method of altering fill material can be at least one of a radiofrequency source, a laser source, a microwave source, a magnetic source and an ultrasound source. Each of these energy sources can be configured to preferentially deliver energy to a cooperating, energy sensitive filler component carried by the fill material. For example, such filler can be suitable chomophores for cooperating with a light source, ferromagnetic materials for cooperating with magnetic inductive heating means, or fluids that thermally respond to microwave energy.

Also disclosed is the use of additional filler materials such as porous scaffold element and materials for allowing or accelerating bone ingrowth. The filler material can comprise reticulated or porous elements of the types disclosed in copending U.S. Patent Application Ser. No. 11/146,891, filed June 7, 2005 , titled "Implants and Methods for Treating Bone". Such fillers also carry bioactive agents. Additional fillers, or the conductive filler, also can include thermally insulative solid or hollow microspheres of a glass or other material for reducing heat transfer to bone from the exothermic reaction in a typical bone cement component.

The above description of the invention is intended to be illustrative and not exhaustive.
The invention also encompasses embodiments as if dependent claims were alternatively written in a multiple dependent claim format with reference to other independent claims. Specific characteristics and features of the invention and methods are described in relation to some figures and not in others, and this is for convenience only.

## Claims

1. A system for treating bone, comprising:
an elongated introducer with a flow channel extending therethrough for introducing a bone fill material into an interior of a bone;
a source of bone fill material coupled to the flow channel and configured to deliver a flow of bone fill material through the flow channel;
an energy source selectively coupleable to the bone fill material to alter the viscosity of the bone fill material;
a sensor configured to measure a characteristic of the bone fill material; and
a controller configured to control the application of energy from the energy source to the bone fill material, wherein the controller includes algorithms that ramp-up and ramp-down power delivery from the energy source to the bone fill material in response to the measured characteristic of the bone fill material.

2. The system for treating bone of Claim 1, wherein the introducer has an electrically and thermally insulative interior sleeve that defines the flow channel.

3. The system for treating bone of Claim 2, wherein the interior sleeve carries conductive surfaces that function as an energy emitter.

4. The system for treating bone of Claims 2 or 3, wherein the interior sleeve is made of a polymer.

5. The system for treating bone of any of the preceding claims, wherein the energy source is operatively connected to an energy emitter.

6. The system for treating bone of Claim 5 wherein the energy emitter comprises spaced apart opposing polarity electrodes.

7. The system for treating bone of any of the preceding claims, wherein controller includes algorithms for adjusting power delivery based on the volume of inflowing bone fill material.

8. A system according to any of the preceding claims, wherein the bone fill material further includes an in-situ hardenable or polymerizable cement component.

9. A system according to Claim 8, wherein the polymerizable component includes at least one of PMMA, monocalcium phosphate, tricalcium phosphate, calcium carbonate, calcium sulphate or hydroxyapatite.

10. The system for treating bone of any of the preceding claims, wherein the controller adjusts at least one delivery parameter selected from volume, pressure, velocity and acceleration of the inflowing bone fill material.

11. The system for treating bone of any of the preceding claims, wherein the controller is configured to calculate viscosity of the bone fill material based on temperature and pressure signals.

12. The system for treating bone of any of the preceding claims, wherein the controller is capable of adjusting energy delivered from the energy source to the inflowing bone fill material in response to impedance, temperature, viscosity feedback or flow parameters to alter viscosity of the bone fill material.

13. The system for treating bone of any of the preceding claims, wherein the viscosity of the fill material is increased to a higher viscosity of at least 1,000,000 cps.

14. The system for treating bone of any of the preceding claims, wherein the energy source selectively increases the viscosity of the bone fill material by at least 100%.

## Patentansprüche

1. System zur Behandlung von Knochen, das Folgendes umfasst:
eine langgestreckte Einbringvorrichtung mit einem sich dadurch hindurch erstreckenden Strömungskanal zum Einbringen eines Knochenfüllstoffs in ein Inneres eines Knochens;
eine Quelle von Knochenfüllstoff, die an den Strömungskanal gekoppelt ist und dazu konfiguriert ist, einen Strom von Knochenfüllstoff durch den Strömungskanal zuzuführen;
eine Energiequelle, die selektiv an den Knochenfüllstoff koppelbar ist, um die Viskosität des Knochenfüllstoffs zu ändern;
einen Sensor, der dazu konfiguriert ist, eine Eigenschaft des Knochenfüllstoffs zu messen; und
einen Regler, der dazu konfiguriert ist, das Anlegen von Energie von der Energiequelle an den Knochenfüllstoff zu regeln, wobei der Regler Algorithmen umfasst, die die Leistungszufuhr von der Energiequelle zu dem Knochenfüllstoff als Reaktion auf die gemessene Eigenschaft des Knochenfüllstoffs hochfahren und herunterfahren.

2. System zum Behandeln von Knochen nach Anspruch 1, wobei die Einbringvorrichtung eine elektrisch und thermisch isolierende innere Hülse aufweist, die den Strömungskanal definiert.

3. System zum Behandeln von Knochen nach Anspruch 2, wobei die innere Hülse leitfähige Oberflächen trägt, die als ein Energieermitter fungieren.

4. System zum Behandeln von Knochen nach Anspruch 2 oder 3, wobei die innere Hülse aus einem Polymer hergestellt ist.

5. System zum Behandeln von Knochen nach einem der vorangehenden Ansprüche, wobei die Energiequelle wirksam mit einem Energieermitter verbunden ist.

6. System zum Behandeln von Knochen nach Anspruch 5, wobei der Energieermitter voneinander beabstandete Elektroden entgegengesetzter Polarität umfasst.

7. System zum Behandeln von Knochen nach einem der vorangehenden Ansprüche, wobei der Regler Algorithmen zum Verstellen der Leistungszufuhr basierend auf dem Volumen einströmenden Knochenfüllstoffs umfasst.

8. System nach einem der vorangehenden Ansprüche, wobei der Knochenfüllstoff weiter eine in-situ aushärtbare oder polymerisierbare Kittkomponente umfasst.

9. System nach Anspruch 8, wobei die polymerisierbare Komponente mindestens eines aus PMMA, Monocalciumphosphat, Tricalciumphosphat, Calciumcarbonat, Calciumsulfat oder Hydroxyapatit umfasst.

10. System zum Behandeln von Knochen nach einem der vorangehenden Ansprüche, wobei der Regler mindestens einen Zufuhrparameter verstellt, der aus Volumen, Druck, Geschwindigkeit und Beschleunigung des einströmenden Knochenfüllstoffs ausgewählt ist.

11. System zum Behandeln von Knochen nach einem der vorangehenden Ansprüche, wobei der Regler dazu konfiguriert ist, die Viskosität des Knochenfüllstoffs basierend auf Temperatur- und Drucksignalen zu berechnen.

12. System zum Behandeln von Knochen nach einem der vorangehenden Ansprüche, wobei der Regler in der Lage ist, die von der Energiequelle zu dem einströmenden Knochenfüllstoff zugeführte Energie als Reaktion auf Impedanz-, Temperatur-, Viskositätsrückmeldung oder Strömungsparameter zu verstellen, um die Viskosität des Knochenfüllstoffs zu ändern.

13. System zum Behandeln von Knochen nach einem der vorangehenden Ansprüche, wobei die Viskosität des Füllstoffs auf eine höhere Viskosität von mindestens 1 000 000 cP erhöht wird.

14. System zum Behandeln von Knochen nach einem der vorangehenden Ansprüche, wobei die Energiequelle die Viskosität des Knochenfüllstoffs selektiv um mindestens 100 % erhöht.

## Revendications

1. Système destiné à traiter les os, comprenant :
un introducteur allongé avec un canal d'écoulement s'étendant à travers celui-ci destiné à introduire un matériau de remplissage osseux à l'intérieur d'un os ;
une source de matériau de remplissage osseux couplée au canal d'écoulement et configurée pour délivrer un écoulement de matériau de remplissage osseux à travers le canal d'écoulement ;
une source d'énergie capable de couplage sélectif avec le matériau de remplissage osseux pour modifier la viscosité du matériau de remplissage osseux ;
un détecteur configuré pour mesurer une caractéristique du matériau de remplissage osseux ; et
un dispositif de commande configuré pour commander l'application d'énergie de la source d'énergie au matériau de remplissage osseux, dans lequel le dispositif de commande inclut des algorithmes qui accélèrent et décélèrent la délivrance de puissance de la source d'énergie au matériau de remplissage osseux en réponse à la caractéristique mesurée du matériau de remplissage osseux.

2. Système destiné à traiter les os selon la revendication 1, dans lequel l'introducteur a une gaine intérieure isolante électriquement et thermiquement qui définit le canal d'écoulement.

3. Système destiné à traiter les os selon la revendication 2, dans lequel la gaine intérieure porte des surfaces conductrices qui fonctionnent comme un émetteur d'énergie.

4. Système destiné à traiter les os selon la revendication 2 ou 3, dans lequel la gaine intérieure est fabriquée dans un polymère.

5. Système destiné à traiter les os selon l'une quelconque des revendications précédentes, dans lequel la source d'énergie est connectée de manière fonctionnelle à un émetteur d'énergie.

6. Système destiné à traiter les os selon la revendication 5 dans lequel l'émetteur d'énergie comprend des électrodes de polarité opposée espacées.

7. Système destiné à traiter les os selon l'une quelconque des revendications précédentes, dans lequel le dispositif de commande inclut des algorithmes destinés à ajuster la délivrance de puissance sur la base du volume du matériau de remplissage osseux affluant.

8. Système selon l'une quelconque des revendications précédentes, dans lequel le matériau de remplissage osseux inclut en outre un composant de ciment durcissable ou polymérisable in situ.

9. Système selon la revendication 8, dans lequel le composant polymérisable inclut au moins l'un parmi le PMMA, le phosphate de monocalcium, le phosphate de tricalcium, le carbonate de calcium, le sulfate de calcium ou l'hydroxyapatite.

10. Système destiné à traiter les os selon l'une quelconque des revendications précédentes, dans lequel le dispositif de commande ajuste au moins un paramètre de délivrance sélectionné parmi le volume, la pression, la vitesse et l'accélération du matériau de remplissage osseux affluant.

11. Système destiné à traiter les os selon l'une quelconque des revendications précédentes, dans lequel le dispositif de commande est configuré pour calculer la viscosité du matériau de remplissage osseux sur la base de signaux de température et de pression.

12. Système destiné à traiter les os selon l'une quelconque des revendications précédentes, dans lequel le dispositif de commande est capable d'ajuster l'énergie délivrée de la source d'énergie au matériau de remplissage osseux affluant en réponse à des retours d'impédance, de température, de viscosité ou à des paramètres d'écoulement pour modifier la viscosité du matériau de remplissage osseux.

13. Système destiné à traiter les os selon l'une quelconque des revendications précédentes, dans lequel la viscosité du matériau de remplissage est augmentée jusqu'à une plus haute viscosité d'au moins 1 000 000 cP.

14. Système destiné à traiter les os selon l'une quelconque des revendications précédentes, dans lequel la source d'énergie augmente sélectivement la viscosité du matériau de remplissage osseux d'au moins 100 %.
